# EUROPEAN PATENT SPECIFICATION

(11) **EP 3 678 500 B1**
(45) Date of publication and mention of the grant of the patent: **10.01.2024**
(21) Application number: 18773548.5
(22) Date of filing: 07.09.2018
(51) Int. Cl.: A24D 1/14, A24F 47/00

(54) **EVAPORATION DEVICES CONTAINING PLANT MATERIAL**
VERDAMPFUNGSVORRICHTUNGEN MIT PFLANZENMATERIAL
DISPOSITIFS D'ÉVAPORATION CONTENANT UNE MATIÈRE VÉGÉTALE

(30) Priority: 07.09.2017 GB 201714412
(43) Date of publication of application: 15.07.2020
(73) Proprietor: Amplicon AB, 754 50 Uppsala (SE)
(72) Inventor: ENGQVIST, Håkan, 752 29 Uppsala (SE)
(74) Representative: Potter Clarkson
(86) International application number: PCT/GB2018/052554
(87) International publication number: WO 2019/048880

(56) References cited:
- WO-A1-2016/142705
- CN-A- 105 755 434
- US-A1- 2015 209 530
- US-A1- 2017 065 000
- US-A1- 2017 239 229

## Description

### Field of the Invention

The invention relates to new inhalation devices that enable a deliverable agent, particularly nicotine, to be delivered in the form of an aerosol or vapour to a user through the use of a porous carrier material containing plant material. Said devices may be useful in effecting delivery of controlled quantities of the deliverable agent to the user.

### Background

The listing or discussion of an apparently prior-published document in this specification should not necessarily be taken as an acknowledgement that the document is part of the state of the art or is common general knowledge.

Active agents may be delivered to recipients through the use of inhalation devices, which evaporate the active agent during use and allow it to be inhaled. Such systems are increasingly being used in the form of e-cigarettes for the delivery of vaporised nicotine to users. See, for example, international patent application WO 99/44448, US patent applications 2014/0202477 and 2014/0014126, and Callahan-Lyon P. *Tob Control;* 2014;23:ii36-ii40.

Evaporation devices typically comprise a chamber containing nicotine in a dissolved or liquid form, together with a heating device for vaporising the liquid. The heating device, which may be a heating element, is generally positioned within the inhalation device so that it can directly heat an evaporation carrier containing a portion of the nicotine, rather than the reservoir of nicotine. The evaporation carrier lies partially submerged in the reservoir and draws the liquid from the reservoir by wicking (capillary action) towards the heating element at which point it is vaporised in order for it to be inhaled.

US patent application 2015/0209530 describes an inhalation device in which the substance to be delivered (either an active pharmaceutical ingredient or nicotine) is provided in the form of a paste before being coated onto a carrier material and allowed to dry. The dried paste and carrier material are then heated within the device to evaporate the active pharmaceutical ingredient or nicotine and allow it to be inhaled by the user. US patent no. 4,303,083 also discloses a device for vaporising a volatile compound through heating the compound in liquid form.

Known inhalation devices, including so-called electronic cigarette ("e-cigarette") devices, often suffer from a number of problems, including providing an unpleasant taste to the user if the device is heated dry (i.e. when the volatile agent has run out). In addition, many of the additives provided in the liquids used therein are harmful and can cause health problems. For example, propylene glycol, vegetable glycerin and polyethene glycol are known to produce compounds such as formaldehyde and acetaldehyde under certain conditions, and such residues may be inhaled as a result of the heating process. Control of dosing is also difficult to achieve which can lead to excessive or imprecise dosing to users.

The handling of large amounts of pure or concentrated nicotine presents a variety of practical difficulties, particularly for end users. Devices, including e-cigarettes, which contain a reservoir of liquid or which must be filled via a connection to an external reservoir can also suffer from problems relating to leakage and spillage of the liquid contents during use and/or filling. There is therefore a need to provide devices in which such difficulties are minimised.

Ceramics are becoming increasingly useful to the medical world, particularly in view of the fact they are durable and stable enough to withstand the corrosive effect of body fluids.

Ceramics are also known to be of potential use as fillers or carriers in controlled-release pharmaceutical formulations. See, for example, EP 947 489 A, US 5,318,779, WO 2008/118096, Lasserre and Bajpai, Critical Reviews in Therapeutic Drug Carrier Systems, 15, 1 (1998), Byrne and Deasy, Journal of Microencapsulation, 22, 423 (2005) and Levis and Deasy, Int. J. Pharm., 253, 145 (2003).

In particular, Rimoli et al, J. Biomed. Mater. Res., 87A, 156 (2008), US patent application 2006/0165787 and international patent applications WO 2006/096544, WO 2006/017336 and WO 2008/142572 all disclose various ceramic substances for controlled release of active ingredients. US 2017/065000 was published on 9 March 2017 and discloses an electrically powered aerosol delivery system for use as a smoking article.

### Disclosure of the Invention

The present invention provides a device for delivering a deliverable agent in the form of an aerosol or vapour to a user according to claim 1, a cartridge or unit dose product for use in an inhalation device according to claim 14, and a method of delivering a deliverable agent in the form of a vapour or aerosol to a user according to claim 15. Further aspects and preferred embodiments are defined in the dependent claims. Aspects, embodiments, examples, and implementations of the present disclosure that do not fall within the scope of the appended claims do not form part of the invention and are merely provided for illustrative purposes.

According to a first aspect of the invention, there is provided a device for delivering a deliverable agent in the form of an aerosol or vapour to a user, comprising a solid, porous carrier material and plant material comprising the deliverable agent, wherein the plant material is located within voids in the carrier material. The device is configured such that it is operable to heat the carrier material and thereby vaporise the deliverable agent.

Devices comprising such features are hereinafter referred to together as "the devices of the invention". They may also be referred to herein as "inhalation devices".

We have advantageously found that devices of the invention provide for release of a deliverable agent (e.g. nicotine, tetrahydrocannabinol or cocaine) in the form of an aerosol (i.e. droplets or liquid or solid in a gas) or a vapour (i.e. a gas) so as to allow a controllable quantity of the deliverable agent to be received by a subject via inhalation. The deliverable agent is a substance (or a mixture of substances) that may be delivered to an individual in order to provide a pleasurable or therapeutic effect. In embodiments of the present invention, the deliverable agent is typically a stimulant (e.g. nicotine) or an analgesic (e.g. a tetrahydrocannabinol or cocaine). Moreover, the plant material is one that contains one or more deliverable agents, such as tobacco, cannabis plant products, or coca plant products.

Once it has been hardened, the carrier material used in the devices of the invention contains voids. Plant material which has been ground, milled or otherwise processed to form a finely divided material is located within these voids during the manufacture of the carrier material, and the size of the voids will be commensurate with the size of the particles of plant material. The average size of the particles of plant material and the voids within which those particles are located is typically from 10 µm to 3000 µm. Preferably the average size of the particles and voids is at least about 50 µm, e.g. at least about 100 µm.

The size of the voids, and the total volume of said voids, in the carrier material should be chosen to enable the carrier material to effectively store a sufficient quantity of the plant material containing the deliverable agent for an extended period of time with minimal loss of the deliverable agent or other volatile components of the plant material under ambient conditions. This can be typically achieved using a carrier material in which the total volume of the voids from about 10% to about 60% of the total volume of the hardened carrier material. In this case, the "total volume of the hardened carrier material" is the volume of the carrier material including all pores and voids contained therein.

Average void sizes may be measured by methods known to the skilled person, for example light optical microscopy, scanning electron microscopy, the mercury intrusion method, the BET (Brunauer, Emmet, and Teller) method, and N₂-adsorption techniques.

As mentioned above, the average size (e.g. the average internal dimension) of the voids is therefore typically from 10 µm to 3000 µm. The plant material that is used in the devices of the invention is provided in a finely divided form in which the average particle size ranges from about 10 µm to about 3000 µm. The particles of plant material become located within the voids in the carrier material during the processed of forming the hardened carrier material structure. Air may be present within each void surrounding the particles of plant material. However, the plant material may alternatively be incorporated into the carrier material in such a way that it is surrounded by a liquid, gel or paste within the voids of the hardened carrier material. Said liquid, gel or paste may itself contain one or more second deliverable agents (which are to be delivered to the user when the device is used) or one or more additional substances mentioned elsewhere herein (e.g. evaporation enhancing agents, flavouring agents, taste enhancers, etc.). For example, said liquid, gel or paste may contain nicotine, particularly when the plant material is tobacco.

The carrier material used in the devices of the invention may also contain pores. In the context of the present invention, these pores are different from, and typically much smaller than, the voids referred to above. For example, the average pore size may be as low as 0.5 nm.

Voids which are located deep within the carrier material, i.e. away from the external surface the carrier material block, may be in gaseous connection with the external surface by way of said pores. The presence of pores linking the inner voids to the external surface enables the deliverable agent contained within those inner voids to be transported to the external surface and subsequently inhaled by the user. The existence of a porous network also enables the carrier material block to act as a filter so that the user of the device does not inhale significant quantities of particulate plant material during use. The porosity must be sufficiently high in order for the carrier material to allow the deliverable agent contained within it to be released in a reasonably short timeframe whilst still being effective as a filter. It is believed that a porosity of at least 10%, preferably at least 30%, (by volume) in the carrier material is sufficient for this purpose. The term "porosity" when used herein refers to ratio of the total volume of open space (including pores and voids) in the carrier material to the total volume of the carrier material block (i.e. the hardened carrier materiel itself together with the pores and voids). The porosity must also be low enough to ensure that carrier material is not too fragile. In a particular embodiment, the carrier material has a porosity of from about 10% to about 80%, such as from about 20% to about 70% (more preferably from about 30% to about 50%). Stronger materials may be suited to having a higher porosity. In embodiments in which the carrier material is based on a ceramic material or a geopolymeric material, the porosity may be from about 20% to about 70%, preferably from about 30% to about 60%, and more preferably from about 30% to about 50%. Control of the porosity of the carrier material is important as this ensures that controlled delivery of the deliverable agent to the user may be achieved.

Control of the pore size in the carrier material is also desirable in order to improve the controlled release characteristics of the devices of the invention. The pore size (e.g. an average internal dimension) of the carrier material should be sufficiently small to avoid rendering the carrier material too weak, and sufficiently large to ensure that the pores can permit transfer of the deliverable agent away from the plant material that is located at the inner regions of the carrier material. In one embodiment of the present invention, the average pore size is not larger than about 10 µm, and is preferably below about 5 µm. The average pore size may be as low as 0.5 nm (for so-called "micropores", as described in the Science of Concrete (http://iti.northwestern.edu/cement/monograph/Monograph7 2.html). Open regions with smaller dimensions are generally classified as interlayer spaces. A typical pore size distribution for hardened cement encompasses a large range, extending from as small as 0.5 nm (or possibly less) to about 10 µm in diameter. The larger pores, ranging from 10 nm to 10 µm, are the residual unfilled spaces between cement grains, and may also be defined as capillary pores. The finest pores range from approximately 0.5 nm to 10 nm. These are often called gel pores since they constitute the internal porosity of (for example) a calcium silicate hydrate gel phase. In one embodiment, the minimum average pore size may be about 0.5 nm, though it may be about 10 nm and is preferably about 0.1 µm. However, in some embodiments, the minimum pore size may be about 0.2 µm, such as about 0.25 µm. Thus in a preferred embodiment, the average pore size in the solid, porous material is from about 0.1 µm to about 10 µm or more preferably from about 0.2 µm to about 5 µm. Average pore sizes may be measured by methods known to the skilled person, for example light optical microscopy, scanning electron microscopy, the mercury intrusion method, the BET (Brunauer, Emmet, and Teller) method, and N₂-adsorption techniques.

It is not necessary for the carrier material to be able to transmit air throughout the entire porous structure. In preferred embodiments, the porosity of the carrier material and/or the average pore size in the carrier material is such that the user is unable to draw a substantial quantity of air through the carrier material pores during inhalation.

Similarly, in particular embodiments, the pore size may be such that airflow within the pores of the carrier material is greatly reduced. The deliverable agent is released from the plant material within the carrier material by heating the carrier material and its contents, and then allowing the deliverable agent to diffuse from the inner regions of the carrier material into the surrounding air. Other volatile substances that are present in the plant material or which are otherwise incorporated into the carrier material may be evaporated at the same time, so that they are also able to diffuse from the inner regions of the carrier material into the surrounding air. Air is drawn over the external surface of the carrier material whereupon it mixes with the vaporised deliverable agent (and other vaporizable substances present) and is carried to the user. The use of the term "external surface" in this context refers to the outermost surface of the solid material, e.g. the outer surface of the pellet, block or disc of carrier material. Such transport mechanisms are particularly important for carrier materials having small average pore sizes, such as not larger than about 10 µm (e.g. not larger than about 5 µm).

In a preferred embodiment, the carrier material has a porosity of from about 10% to about 80% (e.g. from about 20% to about 70%), the average void size in the carrier material is from about 10 to 3000 µm, and the average pore size in the carrier material is from about 0.1 µm to about 10 µm. In a further preferred embodiment, the carrier material has a porosity of from about 20% to about 70%, the average void size in the carrier material is from about 50 µm to 3000 µm, and the average pore size in the carrier material is from about 0.2 µm to about 10 µm.

In a further preferred embodiment, the carrier material has a high mechanical strength (e.g. compressive strength). In this respect, by material of "high mechanical strength" we also include that the structure of that carrier material pore network maintains its overall integrity (e.g. shape, size, porosity, etc.) when a force of about 1 kg-force/cm² (0.098 MPa), such as about 5 kg-force/cm² (0.49 MPa), such as about 7.5 kg-force/cm², e.g. about 10.0 kg-force/cm², preferably about 15 kg-force/cm², more preferably about 20 kg-force/cm², for example about 50 kg-force/cm², especially about 100 kg-force/cm² or even about 125 kg-force/cm² (12.25 MPa) is applied using routine mechanical strength testing techniques known to the skilled person (for example using a so-called "compression test" or "diametral compression test", employing a suitable instrument, such as that produced by Instron (the "Instron Test", in which a specimen is compressed, deformation at various loads is recorded, compressive stress and strain are calculated and plotted as a stress-strain diagram which is used to determine elastic limit, proportional limit, yield point, yield strength and (for some materials) compressive strength)). The mechanical strength is also typically not greater than about 2040 kg-force/cm² (200 MPa), as materials with a very high mechanical strength may have insufficient porosity to enable an adequate quantity of the deliverable agent to be incorporated therein. In embodiments, therefore, the mechanical strength is less than about 200 MPa, preferably less than about 100 MPa.

A particularly preferred carrier material is one in which the size and interconnectivity of the void (and pores, if present) is such that the transmission of significant quantities of air through the voids of the carrier material cannot be achieved through inhalation by the user. By this, we mean that a healthy adult is incapable of inhaling an amount corresponding approximately to an average person's inspiratory capacity (e.g. about 3 litres) through the carrier material in a period of about 20 seconds. Such a carrier material would typically have a porosity of no more than 50%, and/or an average pore size not exceeding about 10 µm (irrespective of the average void size), though such materials could have higher porosities if the average pore size were smaller and *vice versa.* Thus in a particular embodiment, the carrier material has a porosity of up to 50% (e.g. from about 10% to about 50%), and an average pore size of up to about 10 µm (e.g. from about 0.1 µm to about 10 µm). In each case, the average void size is typically from around 10 µm to 3000 µm in order for the voids to contain the plant material. In all of the embodiments described herein, the voids and pores are preferably interconnected to allow deliverable agent (and other vaporizable substances present) to be released from the inner regions of the carrier material (i.e. the regions that are located distally from the external surface of the carrier material). The carrier material should however have sufficient volume of voids, at least in the external regions, to enable a sufficient quantity of plant material (e.g. a sufficient amount of plant material to provide an amount of nicotine equivalent to that which a smoker receives from a single cigarette) to be contained in those voids prior to use.

In an embodiment of the invention, the plant material containing the deliverable agent is located predominantly within the voids of the carrier material. By the use of the phrase *"predominantly within the voids of the carrier material*" it is intended that at least 80% (e.g. at least 90%) by weight of the plant material in the device is located within voids in the carrier material prior to use. In preferred embodiments, substantially all of the plant material in the device is located within voids in the carrier material. By locating the plant material predominantly or essentially completely within the voids of the carrier material, greater control can be achieved over the amount of deliverable agent that is vaporised and delivered to the user during use. As is described elsewhere herein, a preferred embodiment is one which includes an additional quantity of deliverable agent (i.e. in addition to any contained within the plant material in the voids of the carrier material). In a particular embodiment, the device does not comprise a reservoir of deliverable agent that is separate from the carrier material. That is, essentially all of the deliverable agent is located in association with the carrier material, or preferably essentially all of the deliverable agent is located within the pores and voids of the carrier material (whether that deliverable agent is a constituent of the plant material or not).

It is also preferred that the carrier materials used in the devices of the invention are capable of storing and releasing a sufficient quantity of the plant material during use such that it is not necessary for the device to contain an additional reservoir of the deliverable agent. That is, in preferred embodiments, the device does not contain a store of deliverable agent other than that which is located within the pores or which is associated with the plant material located within the voids of the carrier material prior to use.

Porous carrier materials typically contain both open pores and closed pores. The term "open pores" refers to pores (e.g. voids within the material) that are open to the external environment such that, when those pores are otherwise empty, gases in the environment are able to pass in and out of those pores. Such pores are generally located at or close to the surface of the individual carrier material particles. The term "closed pores" refers to pores which are located within particles of carrier material away from the external surfaces, and which may contain material (e.g. gases) which is not able to freely exchange with the external environment.

In embodiments in which the device contains an additional quantity of deliverable agent (i.e. in addition to that contained within the plant material), preferably at least about 70% of the cavity volume of the open pores of the porous carrier material is filled with the deliverable agent (or mixture containing the deliverable agent). In a further such embodiment at least about 90% (e.g. at least about 95%) of the volume of the open pores of the porous carrier material is filled with the deliverable agent (or mixture containing the deliverable agent).

The term "plant material", when used herein, refers to any material that may be obtained from a plant. In this context, the term "plant" includes organisms such as trees, shrubs, herbs, grasses, ferns, and mosses, and may also refer to parts of said organisms. Plant material typically includes cellular matter that is obtained from one or more plant sources. Where the plant material is obtained from a plurality of plant sources, said material may be obtained from a single plant species or multiple plant species. For example, the device (or replaceable cartridge, as described below) may contain multiple types of tobacco. The incorporation of plant material into the device of the invention allows the user of the device to experience a combination of taste and smell that is similar to that which would be experienced if the same plant material were to be conventionally smoked, e.g. in the form of a cigarette. The substances that give rise to these tastes and smells are contained within the plant material prior to heating.

It has been found that plant material can be readily incorporated into the carrier materials described herein. Methods described herein for processing the plant material enable the device to contain whole cells from the plant, and potentially also larger segments of plant tissue, for example where the plant material contains plant leaves or stems that have been milled, ground or shredded. In such processes, the microscopic structure of the plant material remains largely intact at a cellular level, and a substantial proportion of the cells remain whole.

Alternatively, or additionally, the plant material may be processed in such a way that a substantial proportion of the plant cells no longer remain intact. The plant material that is present in the device may therefore comprise plant cell fragments, such as portions of the plant cell wall (e.g. plant cellulose), cytoplasm, chloroplasts and the like. Said plant cell fragments will contain those substances referred to above which, when heated, allow the user to experience a combination of taste and smell that is similar to that which would be experienced if the original plant material were to be smoked in a conventional manner. Thus, in one embodiment, the plant material used in the device comprises plant cell fragments and/or plant cells.

The devices of the invention may, in principle be used with any plant material. In one embodiment, the plant material is obtained from a coca plant, a tobacco plant or a cannabis plant. Said plant material must be processable in such a way that it can be ground, milled, shredded or otherwise processed so that the material is divided into small pieces (e.g. flakes, shards, crumbs or particles) which can be incorporated into the carrier materials described herein. Plant material that is most suited for use in the devices of the invention is material which, when heated or burned, emits or releases one or more substances which can be inhaled to produce a desired therapeutic effect (such as an analgesic effect) or a desired non-therapeutic effect (e.g. to produce a pleasant taste or smell, or to provide a stimulant effect).

Plant materials which are suitable for producing a pleasant taste or smell include products obtained from tobacco plants, such as plants of the Solanaceae (nightshade) family, more specifically the genus *Nicotiana.* Tobacco that is conventionally used in cigarettes, and which may be used in the devices of the present invention, is typically derived from leaves of tobacco plants, particularly from tobacco leaf lamina and tobacco leaf stems. These plant products are usually cured (e.g. in air) before being incorporated into cigarettes, cigars, pipe tobacco, etc. Said plant material is processed by way of a comminution, pulverization or trituration process (e.g. milling, grinding or shredding) so as to produce a particulate form of the material. Said processing methods may also be used to prepare particulate plant material from other plant sources (e.g. from cannabis plant products) for use in the devices of the invention. The tobacco material that is used in the devices of the invention may alternatively or additionally comprise one or more of tobacco dust, tobacco fines and other particulate tobacco by-products formed during, for example, the treating, handling and shipping of tobacco.

Tobacco contains the alkaloid nicotine, and so is capable of providing a stimulant effect when used in the devices of the invention.

The use of tobacco leaves provides a more authentic experience for the user, i.e. the user experiences flavours and aromas that are more consistent with those experienced when smoking a conventional cigarette. The use of devices of the invention containing tobacco leaves rather than a conventional cigarette will be beneficial for the user as the tobacco product will be heated to a lower temperature in the devices of the invention as opposed to a conventional cigarette (in which the tobacco leaves are burned) and so the user is not exposed to the same high quantities of smoke and other unwanted by-products of conventional cigarettes. It has been found that most of the harmful and potentially harmful constituents in cigarette smoke are formed by thermal breakdown of the tobacco when it is burned. The use of devices in which the tobacco is heated and not burned therefore offers the possibility of significantly reducing both the number and the levels of those constituents generated by tobacco products, whilst retaining an acceptable sensory experience for the user. In addition, the product may be more stable and/or less prone to loss of nicotine and other volatile components prior to use.

Inhalation devices containing tobacco (and optionally an additional quantity of nicotine) may be used in the treatment of nicotine dependence, (e.g. nicotine addiction) with a view to aiding an individual in reducing smoking or stopping altogether. Inhalation devices which contain apparatus which is capable of monitoring the usage of the device, and possibly restricting use by the user, may also be particularly advantageous for use in treating nicotine dependence. Such devices may aid the user in recording their usage patterns, and thereby more accurately controlling their usage over time.

Plant materials which are suitable for producing a therapeutic effect (such as an analgesic effect) include products obtained from cannabis plants (such as plants in the family Cannabaceae) and coca plants (such as plants in the family Erythroxylaceae). Cannabis is known to have other medicinal uses. For example, cannabis is used to reduce nausea and vomiting during chemotherapy, to improve appetite in people with HIV/AIDS, and to treat chronic pain and muscle spasms. It also has sedative properties. Cannabinoids are also being investigated for use in treating stroke patients and in treating epilepsy in children. Cannabis plant material that may be used in the devices of the invention includes material known as medical cannabis. This is obtained from cannabis leaves, resin, flowers or flower buds. Medical cannabis is useful in treating conditions such as nausea and vomiting, HIV/AIDS, pain, neurological problems, and post-traumatic stress disorder. The most psychoactive cannabinoid found in the cannabis plant is tetrahydrocannabinol (or delta-9-tetrahydrocannabinol, commonly known as THC). Other cannabinoids include delta-8-tetrahydrocannabinol, cannabidiol (CBD), cannabinol (CBN), cannabicyclol (CBL), cannabichromene (CBC) and cannabigerol (CBG); they have less psychotropic effects than THC, but are said to play a role in the overall effect of cannabis. As is described above in connection with tobacco, said cannabis plant material is processed by way of a comminution, pulverization or trituration process (e.g. milling, grinding or shredding) so as to produce a particulate form of the material. The process comminuting the plant material before it is incorporated into the carrier material helps to ensure that volatilisation of the deliverable agent(s) contained within the plant material occurs sufficiently rapidly during use.

Coca plants (particularly the leaves of such plants) are useful as they contain the cocaine alkaloid which is known to have analgesic and anaesthetic effects. Cocaine is also a powerful nervous system stimulant. Fresh leaves of the coca plant typically contain from about 0.3 to about 1.5% by weight of cocaine. Other alkaloids that may be present include methylecgonine cinnamate, benzoylecgonine, truxilline, hydroxytropacocaine, tropacocaine, ecgonine, cuscohygrine, dihydrocusco-hygrine, nicotine, and hygrine. It is known that plant material obtained from the coca plant, especially when chewed, is able to act as a mild stimulant, and it can also suppress hunger, thirst, fatigue and pain (for example, it may be used to alleviate pain associated with headaches, rheumatism, wounds and sores). It is also known to be useful in alleviating altitude sickness. Devices of the invention in which the plant material contains coca plant material may be similarly useful.

As is described above in connection with tobacco, said coca plant material is processed by way of a comminution, pulverization or trituration process (e.g. milling, grinding or shredding) so as to produce a particulate form of the material. The process of comminuting the plant material before it is incorporated into the carrier material helps to ensure that volatilisation of the deliverable agent(s) (e.g. the cocaine alkaloid) contained within the plant material occurs sufficiently rapidly during use.

The amount of plant material that is provided in the device will depend upon the amount of deliverable agent that is intended to be delivered to the user. The amount of plant material that is present in the carrier material should not be so large as to prevent the carrier material from existing as a solid, hardened mass which is capable of maintaining its macroscopic structure when heated in the device. Preferably the device contains from about 0.5% to about 70%, e.g. from about 1% to about 50%, by weight plant material relative to the weight of the solid, porous carrier material. More preferably, the device may contain from about 2% to about 30% by weight plant material relative to the weight of the solid, porous carrier material.

The device of the present invention is configured such that it is operable to heat the carrier material and thereby vaporise the deliverable agent. The carrier materials, plant materials and deliverable agents described herein may be used in any conventional inhalation device which is configured to deliver one or more substances to a user in the form of an aerosol or vapour (i.e. gas). Such devices would be known to the skilled person and include electronic cigarettes known as "e-Cigs", for example as described in US 2014/0014126, and other inhalation devices, for example as described in US 4,303,083.

Inhalation devices may also be constructed so that they are capable of being used only with the carrier materials and deliverable agents described herein. This may be achieved in a number of ways, e.g. by ensuring that replaceable cartridges (such as those described elsewhere herein) require a specific 3-dimensional shape in order for the carrier material to be heated by the device, or by incorporating conducting material (e.g. iron particles) within the carrier material for induction heating purposes. Additional methods will be known to the skilled person. Such devices are particularly useful as it would then be very difficult for users to use those carrier materials in other devices and, in doing so, lose some control over the amount of deliverable agent that is inhaled.

In an embodiment of the invention, the carrier material containing the substance to be inhaled (i.e. the deliverable agent) is located within the device in gaseous connection with an opening (e.g. a mouthpiece) located on an external surface of the device. When in use, deliverable agent within the carrier material is vaporised, whereupon the vapour flows to the opening and is received by the user (e.g. via a mouthpiece).

The movement of the vapour within the device is typically achieved by the user inhaling at the mouthpiece and thereby drawing the gases out of the inhalation device. The device may also contain a second opening which is in gaseous connection with both the carrier material containing the plant material and the first opening (e.g. mouthpiece) mentioned hereinbefore. This configuration allows the user to draw air through the internal regions of the inhalation device and thereby facilitate the delivery to the user of the vaporised material following generation of those vapours within the device.

In the devices of the present invention, the substance to be inhaled (i.e. the deliverable agent) is typically a solid, a liquid or a gas under ambient conditions. Pure nicotine is typically a liquid under ambient conditions. The inhalation device contains plant materials which acts as a source of the substance to be inhaled (e.g. in a solid or liquid form, or as a dissolved or suspended gas), together with means by which said substance may be volatilised. Suitable means include any heat source which is capable of delivering thermal energy directly to the carrier material in order to vaporise the deliverable agent that is present in the plant material. The deliverable agent is thereby released in the form of an aerosol or a gas (i.e. a vapour). The vaporised material is then delivered to the user, typically by the user inhaling said vapours. Suitable heating apparatuses that may be used to heat the carrier material will be known to the skilled person.

In one embodiment, the carrier material may be directly heated by a flame. In such an embodiment, the device contains a supply of a flammable gas which is capable of being ignited to heat the carrier material.

In a preferred embodiment, the device comprises a heating element (e.g. an electric heating element) which is operable to heat the carrier material and thereby vaporise at least a portion of the deliverable agent that is located within the plant material in the voids of the carrier material. For example, the heating element may be a resistance heater (e.g. in the form of a conducting wire or a heating plate) which releases an effective amount of heat when a current is passed through it. Heating may also occur by way of induction heating. This may be achieved by locating the carrier material in close proximity to a heating element (e.g. a metal object or other conducting structure) which, in turn, may be heated by induction using an electromagnet.

In a further embodiment, the heating element is located proximally to (i.e. in close proximity to, or preferably directly adjacent to) the carrier material. By this, it is meant that the heating element is positioned sufficiently close to the carrier material to allow the heating element to directly heat the carrier material and vaporise the deliverable agent. The heating element may be in direct contact with the carrier material, and furthermore may be intimately mixed into the carrier material. An example of such a heating element is one in which the heating element is a heating coil. Said coil may be wrapped around the outer wall of a block or pellet of carrier material, or it may be embedded within the carrier material mass. Embedding is typically achieved by incorporating the heating element into the mixture of carrier material precursor substances prior to curing or hardening of that mixture, as is described elsewhere herein. The carrier materials used in the devices of the present invention are capable of being heated directly (i.e. rather than via a flow of hot gas originating from the heating element) without degrading and without producing an unpleasant taste for the user. In another embodiment, the heating element is not mixed into the carrier material, but is located adjacent to or distally from the carrier material. In such embodiments, the heating element may be used to heat air which can then be made to flow over and/or through the carrier material containing the deliverable agent in order to vaporise at least a portion of the deliverable agent that is comprised in the plant material in the voids of the carrier material.

Systems in which induction heating is used to heat the carrier material and deliverable agent typically require a metal object or other electrically conducting structure to be present as the heating element in close association with the carrier material and deliverable agent. Discrete particles (e.g. spheres or granules) of a suitable conducting material (e.g. iron or copper) may be dispersed throughout the carrier material to aid in the heating process. The use of such systems allows the device to rapidly end evenly heat the whole volume of carrier material very quickly, and thereby ensure that the amount of deliverable agent that is release is better controlled and more predictable. The suitable conducting material may also be provided in other suitable shapes and geometries, for example, it may be provided as a series of rods, discs or plates, or as a mesh or 3-dimensional network within which the carrier material and plant material may be located. Where the conducting material is dispersed throughout the carrier (e.g. as small particles, rods or a mesh), then typically the amount of conducting material present should be sufficient to ensure that the carrier material can be heated rapidly and thoroughly throughout, and the amount should be low enough to avoid interfering with the efficacy of the ceramic carrier and its contents. Typically, the amount of conducting material (i.e. the heating element) present in the carrier material may be as high as 40% by weight relative to the total weight of the conducting material and carrier material without significantly reducing the mechanical properties of the hardened cement. Preferably, the amount of conducting material present in the carrier material will be no more than 20% by weight relative to the total weight of the conducting material and carrier material. Where the carrier material is formed in contact with only a small number (e.g. less than five, preferably one) larger conducting masses, then the relative amount of conducting material present as the heating element may be much higher, potentially up to 70% (e.g. up to 50%) by weight relative to the total weight of the conducting material and carrier material. In this respect, 3-dimensional networks may be obtained by any conventional method known to the skilled person including 3D printing or foamed metal formation as described elsewhere herein.

The conducting material may also comprise or consist of a ferromagnetic (or ferrimagnetic) material, such as iron. The presence of such magnetic materials can further enhance the heating effects achieved using induction heating because additional heat is generated through magnetic hysteresis losses within the magnetic material. Induction heating is typically able to provide faster heating of the carrier material compared to resistance heaters which are found in conventional e-cigarettes.

Conducting material (e.g. in the form of particles) may also be mixed with the carrier material even in systems which are not intended for induction heating. Irrespective of the method by which the carrier material is heated, the conducting material helps to increase the speed and homogeneity of heat conduction throughout the carrier material to thereby improve the speed and predictability of the evaporation of the deliverable agent.

The carrier material may be housed within an outer casing located within the inhalation device. For example, the carrier material may be housed within a casing which is formed from a thermally conductive material (e.g. a metal such as aluminium or steel) that is capable of storing the carrier material and the deliverable agent when the device is not in use. In such an embodiment, the heating element may also be in direct thermal contact with the external surface of the casing.

Alternatively, the casing may be a ceramic or geopolymeric material, e.g. as defined hereinafter. Preferably, the casing is a ceramic material (either the same as or different from the ceramic carrier described herein) which does not contain any plant material within its pores or voids. Such a ceramic casing provides thermal insulation to the carrier material and deliverable agent contained within it. Ceramic casings are particularly useful in devices of the invention in which carrier materials are heated using induction heating. In these systems, the casing may act as a store for the carrier material containing the heating element (e.g. particles of conducting material) and separate it from the source of alternating magnetic field (e.g. a conducting coil) and from other components of the inhalation device that may be sensitive to high temperatures.

In a further embodiment, a portion of the heating element may be located internally to the carrier material. For example, some or all of the heating element may be at least partially surrounded by the carrier material. In such devices, the carrier material is shaped complementally with the shape of the heating element; that is, the shape of the carrier material fits with the shape of the heating element in order to facilitate a close association between the heating element and the carrier material. This ensures that there is a relatively high area of association between the heating element and the carrier material. The construction of the device in this way allows for a more rapid and efficient heat transfer from the heating element to the carrier material to further aid in controlling of the release of the deliverable agent. "Control of release" may refer to control of the total amount and/or the rate of release of the deliverable agent from the device when in use.

The carrier material may be manufactured *in situ,* i.e. in the presence of the heating element, in order to ensure that the carrier material is shaped complementally with the shape of the heating element. The formation of the carrier material in this way may be achieved in cases where the carrier material is formed from a paste. Said paste is applied to the heating element (which may, for example, be shaped in a coil, grid or straight wire) and then allowed to harden. Alternatively, the carrier material may be provided as a solid which is pre-formed so as to fit with a particular heating element design. For example, the carrier material may be provided as a block of material which optionally contains one or more cavities (e.g. cylindrical bore) into which a heating element may be located once the inhalation device has been assembled. Alternatively, the carrier material may be formed from a paste which is applied to a mould, allowed to harden and then removed from the mould so that it can be incorporated into an inhalation device at a later date. The mould is shaped so that the carrier material solidifies in a form (i.e. shape) that complements the particular heating element design. In embodiments in which the carrier material is provided as a component in a replaceable cartridge (as hereinafter described), pre-formed units of carrier material having a standardised shape may be used in those cartridges. Carrier materials which may be formed at relatively low temperatures (e.g. below 400 °C), such as chemically bonded ceramics and geopolymers, are particularly suited for the manufacture of pre-formed units due to the mouldable properties of the unhardened carrier material mixtures.

Carrier materials which may be formed at relatively low temperatures (e.g. below 400 °C), such as chemically bonded ceramics and geopolymers, are also particularly suited for use with induction heating systems. The conducting material (whether it is in the form of discrete particles or any other structure(s)) may be interspersed throughout the mixture of the carrier material and plant material by introducing the conducting material before the carrier material is hardened or cured. The composite mixture comprising the carrier material (or its precursors), the plant material and the conducting material is typically a paste which can be moulded into any desired shape after the conducting material has been added. The composite can then be hardened without the use of high temperatures which might melt the conducting material. Conversely, conventional sintering processes can involve temperatures which are in excess of 1000 °C and which may melt many metals and degrade the plant material.

The conducting material may alternatively be a 3D network of metal, which is obtainable by methods including 3D printing. The composite mixture comprising the carrier material (or its precursors), the plant material and the conducting material can be obtained by first preparing the 3D network of metal and then incorporating a mouldable ceramic carrier precursor paste which contains both the ceramic carrier precursor(s) and the plant material.

For the avoidance of doubt, the device may comprise plant material together with one or more additional chemical substances, both of which act as sources of deliverable agents (which may be the same or different). Said additional chemical substances may be intended to produce a similar desired therapeutic or non-therapeutic effect to that obtained from the vapours of the heated plant material. Said additional chemical substances are referred to herein as "second deliverable agents" and are provided in a form which is not associated with plant material. That is, the second deliverable agent is not contained within or associated with plant cellular matter. The second deliverable agent is typically a substance that may be extracted from plant material (which plant material may be the same as or different from the plant material used in the device). For example, the device of the invention may contain both tobacco plant material, such as processed tobacco leaves, together with an additional quantity of nicotine, the latter being provided in a form which does not contain plant cells or plant cellular materials. Thus, in one embodiment, the device comprises a quantity of nicotine which is not associated with plant material. In the case of nicotine, it may be provided in the form of a salt, such as nicotine bitartrate. References herein to "nicotine" therefore include references to pharmaceutically-acceptable nicotine salts, unless specified otherwise.

For the avoidance of doubt, where one or more of said second deliverable agents is a component of the plant material (as with nicotine), the reference to an "additional chemical substance" and a "second deliverable agent" includes a second supply of said substance wherein said second supply does not contain plant material.

In devices of the invention which contain nicotine, including those in which plant material is the sole source of nicotine and those which contain both plant material (e.g. tobacco) and a quantity of nicotine which is not associated with plant material, it is preferred that the device contains an amount of nicotine that is at least equivalent to one cigarette, such as from about 8 mg to about 20 mg of nicotine.

As is mentioned hereinbefore, the carrier material used in the devices of the invention may contain pores. It is preferred that, when the device of the invention contains a second deliverable agent, then that second deliverable agent is preferably at least partially located within the pores (and optionally also in the voids) of the carrier material. The second deliverable agent is typically a small molecule substance which is readily capable of penetrating the relatively small pores of the carrier material, whereas the plant material is provided in a form in which the average particle size is substantially larger than the pore diameter, so that the plant material is located almost exclusively within voids in the carrier material (which voids are substantially larger than the pores, as discussed earlier).

In embodiments of the invention in which a second deliverable agent is present, pores of the porous carrier material may be saturated with the second deliverable agent. For the avoidance of doubt, voids in the carrier material also contain plant material as is described elsewhere herein. In devices in which a second deliverable agent is present, optionally together with one or more additional substances mentioned elsewhere herein (e.g. evaporation enhancing agents, flavouring agents, taste enhancers, etc.), then the pores of the porous carrier material may be saturated with said second deliverable agent and any optional additional substances. In this context, the pores that are saturated with the second deliverable agent (or mixture containing the second deliverable agent) include at least the open pores. The closed pores present in the carrier material may or may not also contain the second deliverable agent (or mixture containing the second deliverable agent). It is not necessary for the closed pores to be saturated with the second deliverable agent (or mixture containing the second deliverable agent). By the use of the term "saturated" it is intended that the pores (e.g. at least the open pores) are predominantly filled (e.g. substantially completely filled) with the second deliverable agent (or mixture containing the second deliverable agent), and preferably that the pores contain essentially only the second deliverable agent (or mixture containing the second deliverable agent). These pores should contain a minimal quantity of vacant space (e.g. space that is occupied by atmospheric gases or materials other than the second deliverable agent). For the avoidance of doubt, the devices of the present invention may contain a plurality of second deliverable agents, and references herein to pores which contain essentially only the second deliverable agent also refer to pores which contain essentially only the plurality of second deliverable agents.

In certain embodiments, the device is refillable. In one example, the carrier material, the plant material and the deliverable agent (e.g. deliverable agent that is comprised within the plant material and optionally an amount of a second deliverable agent) may be provided together in a replaceable cartridge. Such a cartridge should be suitable for use in the inhalation devices of the invention as described herein. In such a system, the store of plant material (e.g. tobacco) in the device may be easily replenished by removing a spent cartridge from the inhalation device and replacing it with a full cartridge (i.e. a cartridge which contains the desired quantity of plant material, such as tobacco). Inhalation devices which allow for the replacement of cartridges containing plant material are also refillable devices.

Thus according to a second aspect of the invention, there is provided a cartridge suitable for use in an inhalation device as described herein, wherein the cartridge contains:
(i) a solid, porous carrier material; and
(ii) plant material (e.g. tobacco) comprising a deliverable agent as hereinbefore defined located within voids in the carrier material.

In embodiments of the invention in which the devices are intended for use in the delivery of nicotine, individual units (e.g. in the form of replaceable cartridges as described above, or in the form of blocks, pellets, tablets, discs or sticks as described below) that contain tobacco (and, optionally, an additional quantity of nicotine) and that are suitable for use with these devices may be provided to the end-user. These individual units therefore represent an embodiment of the "cartridge" that is the second aspect of the invention.

The individual units themselves may be supplied to the end-user separately from, or together with, the inhalation device. Each individual unit contains a sufficient amount of nicotine (including that within the plant material optionally together with an additional quantity of nicotine) to provide the desired number of doses to the user, and so may be described as a "unit dose product" or a "controlled dose product". Nicotine may be supplied in a controlled dose product in which each unit (i.e. each pellet, tablet, etc.) contains a sufficient amount of nicotine to provide a plurality of doses (e.g. at least 5, at least 20, or at least 100 doses) when used in the device of the invention. For controlled dose products which contain a plurality of doses, those units may be heated in the devices multiple times over time by the user with each heating event facilitating the delivery of separate dose of nicotine to the user. A single "dose" of nicotine may, for example, correspond to an amount of nicotine equivalent to that which a smoker receives from a single conventional cigarette, or a fraction (e.g. about one tenth or about one fifth) thereof.

Each individual unit (e.g. in the form of a replaceable cartridge as described above, or in the form of a block, pellet, tablet, disc or stick as described below) may typically contain from about 0.5% to about 70%, e.g. from about 1 % to about 50%, by weight plant material relative to the weight of the solid, porous carrier material. Preferably, each unit may contain from about 2% to about 30% by weight plant material relative to the weight of the solid, porous carrier material.

Individual units may also contain both plant material and one or more additional chemical substances, both of which act as sources of deliverable agents. Each unit preferably contains about 0.1% to about 20%, e.g. from about 0.3% to about 10%, by weight deliverable agent relative to the weight of the solid, porous carrier material. More preferably, the device may contain from about 0.5% to about 5% by weight deliverable agent relative to the weight of the solid, porous carrier material. For the avoidance of doubt, the reference to deliverable agent in the context of a unit or device which contains both plant material and one or more additional chemical substances is a reference to the total amount of deliverable agent present from both of these sources combined, unless the contrary is indicated.

In a further embodiment, there is provided a unit product (e.g. a replaceable cartridge, a unit dose product or a controlled dose product) containing:
(i) a solid, porous carrier material;
(ii) plant material, containing a deliverable agent as hereinbefore defined, located within voids in the carrier material; and
(iii) particles of a conducting material (e.g. a metal) distributed throughout the carrier material.

In such an embodiment, the unit product may be used as a replacement for a spent cartridge in an inhalation device as described herein. The carrier material, plant material and conducting material may each be as described elsewhere herein. Such unit products may each contain a defined quantity of the deliverable agent, for example a sufficient amount of the deliverable agent to allow a controlled dose (e.g. no more than about one unit dose) to be delivered to the recipient via inhalation before that unit product is effectively exhausted.

In embodiments in which the carrier material and the deliverable agent are provided together in a replaceable cartridge, unit dose product, controlled dose product, or the like, the unit product may be constructed so that it can be easily removed from the device by the user in order for a replacement unit product (e.g. a replenished cartridge or unit dose product or controlled dose product) to be introduced in its place.

In one embodiment, the replacement cartridge, unit dose product, controlled dose product, or the like may be configured such that the carrier and the plant material are positioned in close proximity to a heating element in the device following insertion of the cartridge, while being simultaneously configured such that the user in unable to come into physical contact with the plant material at any time prior to activation of the device. This may help to reduce the risk of unintended exposure of the user to the deliverable agent when replenishing the device. Alternatively, or additionally, the replacement cartridge, unit dose product, controlled dose product, or the like may contain a composite carrier material, i.e. a material which comprises a carrier material (e.g. a chemically bonded ceramic or geopolymeric material), plant material and particles of a conducting material.

The replacement cartridges may contain the carrier material and the plant material together within a casing as hereinbefore described (e.g. a shell made of a different material from the carrier material, preferably a metal, an alloy, a ceramic or a geopolymer) so as to reduce the exposure of the user to the deliverable agent, or to minimise unintended loss of the deliverable agent during storage or insertion into the device.

In an alternative embodiment, the unit product (e.g. cartridge) may consist essentially of the carrier material, the plant material and the deliverable agent, optionally together with particles of a conducting material and/or one or more additional substances mentioned elsewhere herein that may be present (e.g. evaporation enhancing agents, flavouring agents, taste enhancers, fillers, etc., as would be known to the skilled person). For example, the cartridge may not contain any other elements (aside from the carrier material and the plant material) that are required for the functioning of the device.

The inhalation device may be configured such that, after use, the user simply needs to remove the spent carrier material from the device and insert a replacement unit of carrier material containing a full supply of the deliverable agent (e.g. nicotine). Such replacement units could be provided as blocks, discs, tablets, sticks or pellets of carrier material containing the deliverable agent. Such replacement units could be provided in the commonly used packaging commercially known as a "blister pack" with each unit vacuum packaged, sealed and individually removable for insertion into the device. The provision of such replacement units would minimise wastage and ensure that spare cartridges would be small and could be conveniently stored by the user.

The use of a cartridge system may allow for greater control of the quantity of deliverable agent that is delivered to the user. For example, each cartridge may contain a sufficient quantity of the deliverable agent (e.g. nicotine) to provide a defined amount (e.g. no more than the amount which a smoker receives from a single conventional cigarette, or a fraction (e.g. about one tenth or about one fifth) thereof) of that deliverable agent to the user.

Advantageously, the inhalation devices of the invention may contain more than one carrier material, or a carrier material in which different regions have different average pore sizes. This enables the inhalation devices to be configured so as to release the one or more other deliverable agents (referred to herein as "second deliverable agents") at a plurality of rates. For example, such a device may be able to provide an initial rapid release of a controlled amount of nicotine followed by a slower sustained release of a controlled amount thereof, depending on the needs of the user. Devices of the invention may also be configured to provide an initial rapid release of a nicotine, e.g. from nicotine which is not contained in the plant material, followed by a slower sustained release of vapours derived from the heated plant material. This combination allows the user to obtain the rapid "hit" of nicotine that is desired, without the need to subject the plant material to a heating regime that might disrupt the taste and smell that is provided by volatile products contained therein.

In a preferred embodiment, the carrier material is based on one or more ceramic materials or one or more geopolymeric materials. It is particularly preferred that the carrier material is based on one or more chemically bonded ceramic materials or one or more geopolymeric materials.

The term "ceramic" will be understood to include compounds formed between metallic and nonmetallic elements, frequently oxides, nitrides and carbides that are formed and/or processable by some form of curing process, which often includes the action of heat. In this respect, clay materials, cement and glasses are included within the definition of ceramics (Callister, "Material Science and Engineering, An Introduction" John Wiley & Sons, 7th edition (2007)).

Pore sizes in the carrier material may be controlled by various techniques known to the skilled person. For ceramics (and geopolymers), control of size of pores is typically achieved during the process of fabricating the carrier material network structure. Examples of methods that are known for the fabrication of porous scaffolds are disclosed in Subia B. et al. (2010) Biomaterial Scaffold Fabrication Techniques for Potential Tissue Engineering Applications, Tissue Engineering, Daniel Eberli (Ed.).

A particular method that is suitable for use with the ceramic carrier materials used in the present invention is the porogen leaching method which involves the use of a sacrificial phase during the formation of the carrier material. A porogenic material may be included as part of the reaction mixture during the formation of the carrier material in order to assist in the formation of pores within the final carrier material network. Porogenic materials include, for example, oils, liquids (e.g. water), sugars, mannitol etc. The porogenic material may then be removed from the carrier material, e.g. by dissolving it away using an appropriate solvent, e.g. water.

The carrier material may be based on one or more chemically bonded ceramic materials. These may be provided in the form of granules.

Suitable chemically bonded ceramics include non-hydrated, partly hydrated or fully hydrated ceramics, or combinations thereof.

Non-limiting examples of chemically bonded ceramic systems include calcium phosphates, calcium sulphates, calcium carbonates, calcium silicates, calcium aluminates, magnesium carbonates and combinations thereof. Preferred chemical compositions include those based on chemically bonded ceramics, which following hydration of one or more appropriate precursor substances consume a controlled amount of water to form a network.

Other particular systems available are those based on aluminates and silicates, both of which consume a great amount of water. Phases such CA2, CA, CA3 and C12A7, and C2S and C3S in crystalline or amorphous state (C= CaO, A =Al₂O₃, SiO₂ = S, according to common cement terminology) may be used, which are readily available. The calcium aluminate and/or calcium silicate phases may be used as separate phase or as mixtures of phases. The above-mentioned phases, all in non-hydrated form, act as the binder phase (the cement) in the carrier material when hydrated. The liquid(water)-to-cement weight ratio is typically in the region of 0.2 to 0.5, preferably in the region of 0.3 to 0.4.

Further materials that may be mentioned in this respect include clay minerals such as aluminium silicate and/or aluminium silicate hydrate (crystalline or amorphous). Non-limiting examples include kaolin, dickite, halloysite, nacrite, ceolite, illite or combinations thereof, preferably halloysite.

In further embodiments of the invention, the porous solid is based on a ceramic material that is formed from a self-setting ceramic. Non-limiting examples of self-setting ceramics include calcium sulphate, calcium phosphate, calcium silicate and calcium aluminate based materials. Particular ceramics that may be mentioned in this respect include alpha-tricalcium phosphate, calcium sulphate hemihydrate, CaOAl₂O₃, CaO(SiO₂)₃, CaO(SiO₂)₂, and the like.

Other ceramic materials that may be employed include those based upon a sulphate, such as a calcium sulphate or a phosphate such as a calcium phosphate. Particular examples of such substances include alfa or beta phase calcium sulphate hemihydrate (end product calcium sulphate dihydrate), alkaline or neutral calcium phosphate (apatite) and acidic calcium phosphate (brushite).

The grain size of the ceramic material (e.g. aluminium silicate) may be below about 500 µm, preferably below about 100 µm, more preferably below about 50 µm, and particularly below about 20 µm, as measured by laser diffraction in the volume average mode (e.g. Malvern master size). The use of ceramic material with larger grain sizes may result in a less optimal setting and reduction in the strength of the final solid, though may allow for a better handling of the cement. The grains may be of any shape (e.g. spherical, rounded, needle, plates, etc.). Carrier materials with grain sizes below 1 µm may be used in the devices of the invention, but preferred grain sizes are at least 1 µm, in order to aid with manufacturing (to avoid forming very viscous pastes when wetted), and preferably in the region of about 10 µm. These grain sizes are appropriate for all ceramics in the context of the devices of the invention, including but not limited to chemically bonded ceramics described herein. The grains may be of any shape (e.g. spherical, rounded, needle, plates, etc.). For the avoidance of doubt, where the carrier material is formed from geopolymers, the grain size of the material may similarly be below about 100 µm, more preferably below about 50 µm, and particularly below about 20 µm.

The mean grain size of any ceramic precursor powder particles may be below about 500 µm, e.g. below about 100 µm, preferably between about 1 µm and about 30 µm. This is to enhance hydration. Such precursor material may be transformed into a nano-size microstructure during hydration. This reaction involves dissolution of the precursor material and repeated subsequent precipitation of nano-size hydrates in the water (solution) and upon remaining non-hydrated precursor material. This reaction favourably continues until precursor materials have been transformed and/or until a pre-selected porosity determined by partial hydration using the time and temperature, as well as the H₂O in liquid and/or humidity, is measured.

Chemically bonded ceramics are particularly suitable for use as carrier materials for tobacco. These carrier materials are relatively cheap and easy to manufacture and provide adequate release of the volatile deliverable agent upon the application of heat.

For the avoidance of doubt, the porous solid material may comprise more than one ceramic material, e.g. including a mixture of chemically bonded ceramics.

Pore sizes in chemically bonded ceramics may be controlled by various techniques during the process of fabricating the carrier material network structure. A particular method that is suitable for use with the chemically bonded ceramic carrier materials used in the present invention is the porogen leaching method which involves the use of a sacrificial phase during the formation of the carrier material. A porogenic material may be included as part of the reaction mixture during the formation of the carrier material in order to assist in the formation of pores within the final carrier material network. Porogenic materials include, for example, oils, liquids (e.g. water), sugars, mannitol etc. The porogenic material may then be removed from the carrier material, e.g. by burning it away when the carrier material is heated during the curing process, or by dissolving it away using an appropriate solvent. Dissolving is usually achieved with water in order to avoid leaving residual amounts of a substance which may have deleterious effects on the working of the device or adverse effects on the user.

Foaming methods may also be used to increase the pore sizes in chemically bonded ceramics, as well as other carrier materials mentioned herein. Such methods would be known to the skilled person and are particularly useful for forming carrier materials with larger pore sizes.

Alternatively, the carrier material may be based on one or more geopolymer materials.

The term "geopolymer" will be understood by those skilled in the art to include or mean any material selected from the class of synthetic or natural aluminosilicate materials which may be formed by reaction of an aluminosilicate precursor material (preferably in the form of a powder) with an aqueous alkaline liquid (e.g. solution), preferably in the presence of a source of silica.

The term "source of silica" will be understood to include any form of a silicon oxide, such as SiO₂, including a silicate. The skilled person with appreciate that silica may be manufactured in several forms, including glass, crystal, gel, aerogel, fumed silica (or pyrogenic silica) and colloidal silica (e.g. Aerosil).

Suitable aluminosilicate precursor materials are typically (but not necessarily) crystalline in their nature and include kaolin, dickite, halloysite, nacrite, zeolites, illite, preferably dehydroxylated zeolite, halloysite or kaolin and, more preferably, metakaolin (i.e. dehydroxylated kaolin). Dehydroxylation (of e.g. kaolin) is preferably performed by calcining (i.e. heating) of hydroxylated aluminosilicate at temperatures above 400°C. For example, metakaolin may be prepared as described by Stevenson and Sagoe-Crentsil in J. Mater. Sci., 40, 2023 (2005) and Zoulgami et al in Eur. Phys J. AP, 19, 173 (2002), and/or as described hereinafter. Dehydroxylated aluminosilicate may also be manufactured by condensation of a source of silica and a vapour comprising a source of alumina (e.g. Al₂O₃).

Thus in a further embodiment, the carrier material may be a material obtainable by the process of reacting an aluminosilicate precursor material, such as a material selected from the group consisting of kaolin, dickite, halloysite, nacrite, zeolites, illite, dehydroxylated zeolite, dehydroxylated halloysite and metakaolin, with an aqueous alkaline liquid, optionally in the presence of a source of silica.

Precursor substances may also be manufactured using sol-gel methods, typically leading to nanometer sized amorphous powder (or partly crystalline) precursors of aluminosilicate, as described in Zheng et al in J. Materials Science, 44, 3991-3996 (2009). This results in a finer microstructure of the hardened material. (Such as sol-gel route may also be used in the manufacture of precursor substances for the chemically bonded ceramic materials hereinbefore described.)

If provided in the form of a powder, the mean grain size of the aluminosilicate precursor particles are below about 500 µm, preferably below about 100 µm, more preferred below about 30 µm.

In the formation of geopolymer materials, such precursor substances may be dissolved in an aqueous alkaline solution, for example with a pH value of at least about 12, such as at least about 13. Suitable sources of hydroxide ions include strong inorganic bases, such as alkali or alkaline earth metal (e.g. Ba, Mg or, more preferably, Ca or, especially Na or K, or combinations thereof) hydroxides (e.g. sodium hydroxide). The molar ratio of metal cation to water can vary between about 1:100 and about 10:1, preferably between about 1:20 and about 1:2.

A source of silica (e.g. a silicate, such as SiO₂) is preferably added to the reaction mixture by some means. For example, the aqueous alkaline liquid may comprise SiO₂, forming what is often referred to as waterglass, i.e. a sodium silicate solution. In such instances, the amount of SiO₂ to water in the liquid is preferably up to about 2:1, more preferably up to about 1:1, and most preferably up to about 1:2. The aqueous liquid may also optionally contain sodium aluminate.

Silicate (and/or alumina) may alternatively be added to the optionally powdered aluminosilicate precursor, preferably as fume silica (microsilica; AEROSILO silica). The amount that may be added is preferably up to about 30 wt%, more preferably up to about 5 wt% of the aluminosilicate precursor.

The presence of free hydroxide ions in this intermediate alkaline mixture, causes aluminium and silicon atoms from the source material(s) to be dissolved. The geopolymer materials may then be formed by allowing the resultant mixture to set (cure or harden), during which process the aluminium and silicon atoms from the source materials reorientate to form a hard (and at least largely) amorphous geopolymeric material. Curing may be performed at room temperature, at elevated temperature or at reduced temperature, for example at around or just above ambient temperature (e.g. between about 20°C and about 90°C, such as around 40°C). The hardening may also be performed in any atmosphere, humidity or pressure (e.g. under vacuum or otherwise). The resultant inorganic polymer network is in general a highly-coordinated 3-dimensional aluminosilicate gel, with the negative charges on tetrahedral Al³⁺ sites charge-balanced by alkali metal cations.

In this respect, a geopolymer-based carrier material may be formed by mixing a powder comprising the aluminosilicate precursor and an aqueous liquid (e.g. solution) comprising water, a source of hydroxide ions as described hereinbefore and the source of silica (e.g. silicate), to form a paste. The ratio of the liquid to the powder is preferably between about 0.2 and about 20 (w/w), more preferably between about 0.3 and about 10 (w/w). Calcium silicate and calcium aluminate may also be added to the aluminosilicate precursor component.

In a preferred embodiment of the invention, the plant material is co-formedly interspersed in voids in the carrier material network. This means that, whatever process is employed to form the carrier material, it must also necessarily form voids within which the particulate plant material is interspersed. Carrier material which is based on one or more chemically bonded ceramic materials or one or more geopolymeric materials is particularly suited for use in such embodiments as the process by which the carrier material and its pore network is formed does not require very high temperatures, in contrast to sintered ceramics.

The plant material (and optionally a second deliverable agent) may thus be mixed with the carrier material (e.g. the ceramic or geopolymer) or precursor(s) thereto, by way of a variety of techniques, such as introduction by way of a sol-gel process, as a solution, or as a slurry, a paste or a putty of, for example, particles, granules or pellets of carrier material or precursor(s) thereto, in the presence of an appropriate liquid (e.g. an aqueous or organic solvent). This is followed by some sort of "curing" process to form the sustained release composition, which comprises said voids, within which the plant material resides. Carrier materials that are formed in this way may be said to be pre-loaded with the plant material (e.g. tobacco) containing the deliverable agent.

Such voids are themselves a three-dimensional network of channels or voids within the solid network, containing (e.g. particles of) the plant material.

Such voids may thus be essentially "secondary pores" formed by chemical interactions (e.g. "bonding") between the surfaces of primary particles of carrier material (which may be porous in their own right (i.e. comprise "primary" pores)), such as ceramics or geopolymers. Such pores may, for example, result from exposure of such materials to one or more chemical reagents that cause a physical and/or chemical transformation (such as a partial dissolution) at, and subsequent physical and/or chemical bonding together of, those surfaces (which may in itself result as a consequence of some other physico-chemical process such as drying, curing, etc.), giving rise to said pores/voids.

In such instances, such chemical reagents may be mixed together with the plant material (and optionally a second deliverable agent) during preparation of the carrier material. However, such secondary pores are not necessarily formed in this way, and bonding together of primary particles of carrier materials may also be physical and/or mechanical, or may be formed during the production of a three-dimensional, chemically bonded ceramic network as described hereinbefore, in the presence of the plant material.

Thus, a device for delivering a deliverable agent in the form of an aerosol or vapour to a user is provided, comprising a carrier material which is a solid, continuous three-dimensional network comprising particles of a ceramic material, which particles are bonded together to form secondary pores or voids, and plant material comprising a deliverable agent present within said secondary pores or voids.

Alternatively, if the network is formed by way of a chemical reaction (e.g. polymerisation, or as described hereinbefore for geopolymers), plant material comprising deliverable agent may be co-mixed with a precursor mixture comprising relevant reactants and thereafter located within pores or voids that are formed during formation of the three-dimensional carrier material network itself.

It is particularly preferred that the ceramic material is one that is based on a chemically bonded ceramic or a geopolymer, as these materials are particularly suited for facilitating loading of the tobacco before the pore network is formed in the carrier. This, in turn, offers an effective method for readily controlling the amount of nicotine that is loaded into the carrier during manufacture.

For geopolymers, control of size of pores is typically achieved during the process of fabricating the carrier material network structure. Examples of methods that are known for the fabrication of porous scaffolds are disclosed in Subia B. et al. (2010) Biomaterial Scaffold Fabrication Techniques for Potential Tissue Engineering Applications, Tissue Engineering, Daniel Eberli (Ed.).

A particular method that is suitable for use with the geopolymeric carrier materials used in the present invention is the porogen leaching method described above in respect of the ceramic carrier materials. Porogenic materials that may be used in the formation of porous geopolymeric material include, for example, oils, liquids (e.g. water), sugars, mannitol etc.

Any of the carrier materials described herein may be used in the devices of the invention. Thus, in a further embodiment, the invention relates to a device as hereinbefore described in which the ceramic material is selected from the list consisting of:
(i) a material obtainable by the process of reacting an aluminosilicate precursor material with an aqueous alkaline liquid; and
(ii) a calcium phosphate, a calcium sulphate, a calcium carbonate, a calcium silicate, a calcium aluminate, a magnesium carbonate, an aluminium silicate, and combinations thereof.

We have advantageously found that devices of the invention provide for release of the deliverable agent in the form of an aerosol or a vapour such that the deliverable agent can be administered to a user via inhalation. When in use, the inhalation device allows a deliverable agent to be inhaled by the user, typically for recreational use.

The deliverable agent may be provided in the device as part of a mixture comprising suitable plant material and one or more additional components. One or more of said additional components may be present to facilitate the volatilisation of the deliverable agent when the carrier material is heated. In the inhalation devices of the present invention, the carrier material may have a porosity which aids in controlling the quantity and/or rate of delivery of the deliverable agent received by the user.

The deliverable agent may therefore be provided in a mixture containing one or more evaporation enhancing agents, i.e. agents which enhance the vapour formation of a vapour of the deliverable agent. Suitable evaporation enhancing agents include glycerin, vegetable glycerin (VG), propylene glycol, polyethylene glycol or mixtures thereof.

However, the devices of the invention may advantageously afford a method by which the deliverable agent may be delivered to the user without the requirement for evaporation enhancing agents, such as those above, which may potentially be toxic or which may degrade during the heating process to form toxic by-products. Thus, in a preferred embodiment, the plant material containing the deliverable agent is provided (in the carrier material) either alone or in a mixture which does not contain any of the above-mentioned evaporation enhancing agents.

In another embodiment, the deliverable agent may be provided in a mixture containing one or more additional substances which are not intended to provide any therapeutic benefit to the user. By way of example, said additional substances may be present in order to aid in the manufacture of the product, to aid in the vaporisation of the deliverable agent, or to improve the experience for the user.

The aerosol or vapour that is delivered to the user consists essentially of air, the deliverable agent (e.g. nicotine or THC) and potentially one or more optional additional substances (e.g. an evaporation enhancing agent) that may be present in admixture with the deliverable agent. For example, the aerosol or vapour may also contain any desired flavouring agent (e.g. a flavouring or sweetener as described herein) or inert additive for improving the taste, consistency or texture of the aerosol or vapour, thereby making the inhalation more palatable to the user (i.e. the smoker). However, for devices which contain tobacco, preferably no additional flavouring agent or other such additive is included in the carrier material in order to avoid affecting or detracting from the taste experience that is obtained through heating the tobacco itself.

In one embodiment, the deliverable agent is nicotine. Typically, inhalation devices containing nicotine will be used by smokers as a combustion-free alternative to cigarettes, cigars and pipes, thereby reducing exposure to many of the potentially toxic components found in those tobacco products. Such a combustion-free device is commonly referred to as an "electronic cigarette", "smokeless cigarette," "e-cigarette" or "e-cig". Nicotine is typically obtained from tobacco products, e.g. tobacco oil and other extracts, and is usually present in such products as nicotine bitartrate. Both nicotine and nicotine bitartrate may be used in the inhalation devices described here.

Inhalation devices of the present invention which contain nicotine may be used to produce a pleasurable effect in the user.

Inhalation devices which contain apparatus which is capable of monitoring the usage of the device, and possibly restricting use by the user, may also be particularly advantageous. Such devices may aid the user in recording their usage patterns, and thereby more accurately controlling their usage over time.

The deliverable agent that is to be delivered to the user should be a heat-stable substance, preferably one which is stable at temperatures up to at least about 400°C, more preferably up to at least about 600°C. By the use of the term "heat-stable", it is meant that the deliverable agent is sufficiently stable at that temperature to ensure that it would not undergo significant chemical degradation during use, e.g. when the deliverable agent is a heat-stable pharmaceutical substance then "heat stable" refers to pharmaceutical substances which exhibit not more than 5% degradation when heated to 200°C for 30 seconds.

Nicotine may further be employed in salt form or any other suitable form, such as e.g. a complex or solvate thereof, or in any physical form such as, e.g., in an amorphous state, as crystalline or part-crystalline material, as co-crystals, or in a polymorphous form, or a combination of any of the above.

Pharmaceutically-acceptable salts of nicotine that may be mentioned include acid addition salts and base addition salts. Such salts may be formed by conventional means, for example by reaction of a free base form of nicotine with one or more equivalents of an appropriate acid, optionally in a solvent, or in a medium in which the salt is insoluble, followed by removal of said solvent, or said medium, using standard techniques (e.g. *in vacuo,* by freeze-drying or by filtration).

Examples of pharmaceutically-acceptable addition salts include those derived from mineral acids, such as hydrochloric, hydrobromic, phosphoric, metaphosphoric, nitric and sulphuric acids; from organic acids, such as succinic acid, and particularly tartaric, acetic, citric, malic, lactic, fumaric, benzoic, glycolic, gluconic, succinic, arylsulphonic acids; and from metals such as sodium, magnesium, or preferably, potassium and calcium. In embodiments in which the deliverable agent is nicotine, a particular salt that may be mentioned is nicotine bitartrate. References herein to "nicotine" include references to pharmaceutically-acceptable nicotine salts such as nicotine bitartrate, unless specified otherwise.

The devices of the invention may also be configured to allow the user to monitor and/or record their usage of the device over an undefined period of time. This may aid the user and medical professionals in accurately recording the amount and frequency at which the deliverable agent (e.g. the nicotine) has been administered to the user.

In a further embodiment, the devices of the invention may comprise apparatus for recording the usage history of the device over a defined period of time. Such apparatus may comprise an electronic device which records the relevant events such as the total number of uses of the device, the number of times that the device has been refilled (e.g. the number of times that a cartridge has been replaced), the times at which the device is used or refilled, the amount of deliverable agent that has been delivered to the user, and the like. Such data may be stored on the device so that it may be downloaded to a separate data processing device by the user or a medical professional, or the device may further comprise a display unit so that the data may be visually displayed.

In a further embodiment, the device may comprise an interface or a data transmission unit to allow the recorded data to be extracted from the device for separate analysis. A suitable interface includes a Universal Serial Bus (USB) or another similar component allowing an electrical connection suitable for data transfer. In devices which contain a data transmission unit, said data may be transmitted by the device, e.g. via Bluetooth or similar, to a separate device. In each case, the separate device may be an electronic data processing device which contains suitable software (e.g. an app) for processing the data received from the inhalation device.

In a yet further embodiment, the device may be configured to monitor the usage by the user, and optionally control the extent to which the deliverable agent is administered to the user. For example, the device may be configured so that the number of times that it may be used in a specific period may be restricted. This is particularly useful where it is inadvisable for the user to be able to receive a large quantity of the deliverable agent over a given period of time. Such devices may therefore allow metering of the amount of deliverable agent that is being administered to the user.

Protective coatings may also be used in conjunction with the carrier materials of the presently disclosed devices.

Protective coatings may be used to help control the rate of vaporisation of the deliverable agent during use. One or more coatings may be applied to the external surface of the carrier material. When the carrier material is heated during use, the coating may help to control the temperature at which evaporation occurs. This, in turn, may further aid in controlling the delivery of the deliverable agent, for example by ensuring that the user receives the vaporised material in a short period of time and thereby reducing the likelihood that the user may cease inhaling before having received the entire intended dose.

Protective coatings may also be useful in improving the stability of the deliverable agents within the device. For example, the coating may shield the one or more of the deliverable agents from the external environment, or it may act as a barrier between deliverable agents thereby reducing the extent to which they may mix and chemically interact with each other.

The carrier material that is used in the devices of the invention may be designed to be inert in the following ways:
(a) general physico-chemical stability under normal storage conditions, including temperatures of between about minus 80 and about plus 50°C (preferably between about 0 and about 40°C and more preferably room temperatures, such as about 15 to about 30°C), pressures of between about 0.1 and about 2 bars (preferably at atmospheric pressure), relative humidities of between about 5 and about 95% (preferably about 10 to about 75%), and/or exposure to about 460 lux of UV/visible light, for prolonged periods (i.e. greater than or equal to six months). Under such conditions, carrier material networks as described herein may be found to be less than about 5%, such as less than about 1% chemically degraded/decomposed, as above; and
(b) general physico-chemical stability under acidic, alkaline and/or alcoholic (e.g. ethanolic) conditions at room temperature and/or under at elevated temperatures (e.g. up to about 200°C), which may result in less than about 15% degradation, so avoiding the possibility of deliberate ex *vivo* extraction of the nicotine.

It is preferred in this respect that the network exhibits a compressive strength of greater than about 1 MPa, such as greater than about 5 MPa, e.g. about 10 MPa on micro- and nano-structure level, which is high enough to withstand breakdown of the material at the microstructure level, i.e. of less than about 200 µm.

The plant material that is used in the device of the invention may be obtained from any conventional sources. When the plant material is a tobacco product, that product may be obtained by taking parts of the tobacco plant (leaf, stem, and the like) and grinding the dried portions into a fine powder. Tobacco dust or tobacco fines, which are by-products of certain tobacco processing methods may also be used. The tobacco parts used to make the tobacco product can originate from any type of tobacco used to prepare oral tobacco products such as but not limited to Burley, Bright, Oriental, or blends thereof, as well as genetically altered, chemically altered, or mechanically altered tobacco plants and blends thereof. The starting materials may comprise any part of the tobacco plant, that is unaged or aged leaf, stem or stalk portions or mixtures thereof,

Incorporation of plant material into the carrier material can be facilitated by dividing the plant material in small pieces (e.g. flakes, shards, crumbs or particles) using any conventional method that would be known to the skilled person. Such methods include grinding, milling, shredding and cutting, e.g. with scissors, shears or a cutting mill. The finely divided plant material may then be incorporated into the carrier material using the methods described hereinafter. It is preferred that the finely divided plant material comprises small pieces of plant matter which are not more than 3 mm in size. The process of finely dividing the plant material is monitored and controlled by way of conventional methods for preparing powders in the pharmaceutical industry (see, for example, Patel, R. P., et al., Asian Journal of Pharmaceutics, 2008 Oct-Dec, 216-220). Suitable monitoring methods are known to those skilled on the art, and include sieve analysis, light microscopy and laser diffraction. Control of the particle size of the plant material is important for content uniformity, and it may facilitate mixing and drying during formulation. The use of more finely divided plant material increases the surface area of that component and thereby facilitates the volatilization of the deliverable agent therein.

The ceramic material containing the plant material and the deliverable agent may be prepared by way of a variety of routine techniques, and using standard equipment, known to the skilled person, including mixing together the deliverable agent and the carrier material or its precursors.

Standard mixing equipment may be used for mixing together components of compositions described herein. The mixing time period is likely to vary according to the equipment used, and the skilled person will have no difficulty in determining by routine experimentation a suitable mixing time for a given combination of ingredient(s).

The plant material (e.g. tobacco) may be mixed with the carrier material (e.g. ceramic) by way of a variety of techniques, such as introduction by way of a sol-gel process, as a solution, a slurry, a paste or a putty. The introduction of the mixture comprising the plant material and a carrier material (or precursor(s) thereto) may be followed by some sort of "curing" to form the voids in which the deliverable agent resides. It is during this process that the porous carrier material network may be formed.

A preferred process for the formation of carrier material for use in devices of the invention involves the mixing together of a carrier material (e.g. a ceramic material or precursor(s) thereto) and plant material, and then adding a liquid, such as an aqueous solvent (e.g. water), so providing a wet granulate.

Another preferred process for the formation of carrier material for use in devices of the invention involves the mixing together of the plant material with an aqueous solvent (e.g. water), before combining this mixture with a carrier material (e.g. a ceramic material or precursor(s) thereto).

Wet granulation techniques are well known to those skilled in the art and include any technique involving the massing of a mix of dry primary powder particles using a granulating fluid, which fluid comprises a volatile, inert solvent, such as water, optionally in the presence of a pelletisation aid material.

The product obtained by the above-mentioned process may further be adapted by:
(I) extrusion of the granulate (in cases where granulation takes place);
(II) spheronisation (forcing a wet mass through a sieve to produce pellets);
(III) drying; and/or
(IV) (if necessary) hardening by way of heat,
using routine techniques in all cases.

In the process for formation of carrier materials comprising geopolymers for use in devices of the invention, preformed geopolymer may be reacted together with further aluminosilicate precursor and aqueous alkaline liquid (e.g. solution), preferably in the presence of a source of silica (as hereinbefore described), also in the presence of the plant material as hereinbefore described. For compositions of the invention comprising geopolymers, curing may be performed by allowing the resultant mixture to harden into any given shape, e.g. blocks, pellets, granules or a powder. In this respect, the mixture may be transferred into moulds and left to set as pellets/granules or alternatively (e.g. preferably) pellets/granules may be manufactured using an appropriate extrusion-spheronisation technique. Here, the formed paste (powder and liquid mixture) may be extruded through an orifice. The size of the orifice may be from about 10 µm up to about 30 mm, preferably from about 100 µm to about 1 mm. If larger pellets/granules are required, the size of the orifice may be larger, e.g. from about 1 mm up to about 30 mm, or preferably from about 1 mm up to about 10 mm. The formed extrudate may then be placed in a spheroniser, which is typically a vertical hollow cylinder with a horizontal rotating disk located inside. When the disk is spun, the extrudate is broken into uniform lengths and gradually formed into spherical pellets, which may then be left to harden as described hereinbefore.

In all cases, suitable pellet/granule sizes are in the range of about 0.05 mm to about 3.0 mm (e.g. about 2.0 mm, such as about 1.7 mm), and preferably about 0.1 mm (e.g. about 0.2 mm) to about 1.6 mm (e.g. about 1.5 mm), such as about 1.0 mm.

Carrier materials for use in devices of the invention may further comprise one or more further commonly-employed pharmaceutical excipients. Suitable excipients include inactive substances that are typically used as a carrier for active pharmaceutical ingredients in medications. Suitable excipients also include those that are employed in the pharmaceutical arts to bulk up pharmaceutical compositions that employ very potent active pharmaceutical ingredients, to allow for convenient and accurate dosing. Alternatively, excipients may also be employed in manufacturing processes of the compositions of the invention to aid in the handling of the plant materials and ceramic materials concerned.

In this respect, pharmaceutically-acceptable excipients include filler particles, by which we include particles of material that do not take participate chemically in the process during which the carrier material that is used in the devices of the invention is formed. Such filler particles may be added as ballast and/or may provide the composition with functionality. Non-limiting examples include: zirconium dioxide and barium sulfate to increase radio-opacity, which may be added to smaller particles (e.g. milled) of carrier material used in the devices of the invention. The amount of added filler particles may be up to about 80 wt%, preferably up to about 40 wt% of the weight of the carrier material. Preferably the total volume of the filler is relatively small (e.g. below about 50% by volume of the entire carrier material structure (including pores)) in order to ensure that the carrier material retains a sufficient mechanical strength.

Additional pharmaceutically-acceptable excipients include carbohydrates and inorganic salts such as sodium chloride, calcium phosphates and calcium carbonate.

The carrier material may alternatively be milled to a fine powder, preferably with a powder grain size of below about 100 µm, and more preferably below about 20 µm. Carrier materials with grain sizes below 1 µm may be used in the devices of the invention, but preferred grain sizes are in the region of about 10 µm. Milling is optionally performed using ball-milling, planetary ball-milling, jet milling or a combination thereof.

In the aforementioned embodiments, the carrier material may further include a pelletisation aid material. A pelletisation aid material may be defined as a material that is capable of controlling the distribution of granulating liquid through the wet powder mass during pelletisation and to modify the rheological properties in the mixture. Suitable pelletisation aid materials include hydroxypropylmethylcellulose (HPMC), hydroxyethylcellulose (HEC) and, preferably, microcrystalline cellulose. If present, the pelletisation aid material is preferably employed in an amount of between 0.5 and 50% by weight based upon the total weight of the tablet. A preferred range is from 1 to 20%, such as from about 2.0 to about 12% (e.g. about 10%) by weight.

The carrier material used in the devices of the invention may also optionally contain bulking agents, porogens, dispersion agents or gelating agents to control the rheology and porosity. The total amount of such excipients is limited to about 20 wt% of the total weight of the carrier material (i.e. the ceramic or geopolymeric material) including any other components that may be present (e.g.nicotine, bulking agents, etc.). Non-limiting examples of such excipients include polycarboxylic acids, cellulose, polyvinylalchol, polyvinylpyrrolidone, starch, nitrilotriacetic acid (NTA), polyacrylic acids, PEG, sorbitol, mannitol, glycerol, pharmaceutically-acceptable oils (including vegetable oils (olive oil, maize oil, corn oil, peanut oil, sunflower oil, flaxseed oil, palm oil, castor oil, soybean oil, etc.), essential oils (e.g. evening primrose oil), omega 3 oils (e.g. fish oils), paraffin oil, lipid oils derived from animal issue, silicone oils, etc.), and combinations thereof. Liquid excipients (such as glycerol) may be used in e-liquids for a number of reasons. For example, the liquid may act as a solvent for the nicotine, and it may also help to produce thicker clouds for the user. If a user wants to have more visible aerosol, this may be achieved by increasing the relative amount of the liquid (such as glycerol) that is contained within the carrier material in the device. However, the viscous properties of liquids such as glycerol may thicken the liquid and affect the release profile. Combining the first liquid with an alcohol (such as propylene glycol or ethanol) which has lower viscosity can minimise such difficulties.

The carrier material may also comprise one or more binders. A binder may be defined as a material that is capable of acting as a bond formation enhancer, facilitating the incorporation of the plant material into the carrier material. Suitable binders include cellulose gum and microcrystalline cellulose. If present, binder is preferably employed in an amount of between 0.5 and 20% by weight based upon the total weight of the carrier material and the materials contain therein. A preferred range is from 1 to 15%, such as from about 2.0 to about 12% (e.g. about 10%) by weight.

The carrier material may also comprise one or more taste masking agents, flavourings (e.g. lemon, peppermint powder or, preferably, menthol), or sweeteners (e.g. neohesperidin, acesulfame K or, preferably, sucralose).

The carrier materials may also comprise one or more colourants (e.g. iron oxide for red, cobalt for blue, titanium oxide for white, and so forth). Those colourants would typically be provided in the form of particles of said coloured material with an appropriate size to enable the colour to be visible without significantly affecting the ability of the carrier material to store the deliverable agent and release it upon heating. As with all of the additives discussed above, the particles of colourant may be added to the mixture of ceramic precursor materials before that mixture is cured or hardened.

The devices of the present invention may be used to deliver one or more deliverable agents to a user. Thus, in a third aspect of the invention there is provided a method of delivering a deliverable agent in the form of a vapour or aerosol to a user, which method comprises:
providing an article comprising:
   (i) a solid, porous carrier material as defined herein; and
   (ii) plant material comprising a deliverable agent as defined herein located within voids in the carrier material; and
heating the carrier material to vaporise the deliverable agent.

In one embodiment, the article is an inhalation device as defined herein. In an alternative embodiment, the article is a cartridge or unit product that is suitable for use in an inhalation device as defined herein. Thus, in a fourth aspect of the invention there is provided an article as defined herein. In embodiments in which the article is a cartridge that is suitable for use in an inhalation device as defined herein which contains a heating element, it is preferred that the cartridge is shaped to fit with the heating element.

In a further embodiment, there is provided the use of the article as defined above in the delivery of a deliverable agent in the form of a vapour or aerosol to a user. Similarly, said use will involve the step of heating the carrier material in order to vaporise the deliverable agent to allow it to be inhaled by the user.

In particular embodiments, the methods and uses defined hereinbefore may involve the administration of the deliverable agent to the user for non-therapeutic purposes (e.g. for pleasure (i.e. recreational use)). A particular example of such a method or use is one in which the plant material is tobacco. Devices which contain coca plant material or cannabis plant material may also be used for recreational purposes (instead of or in addition to being used for therapeutic purposes).

In other particular embodiments of the third aspect of the invention, particularly in embodiments in which the device is configured to monitor the usage of the device by the user, and optionally control the extent to which the deliverable agent may be administered to the user, the method may involve the administration of a controlled dosage of the deliverable agent to the user. This is particularly useful where the deliverable agent is addictive or toxic.

In a further aspect of the invention there is provided a method of treating nicotine dependence (e.g. nicotine addiction) wherein the method involves using an inhalation device of the present invention in which the plant material is tobacco to deliver nicotine in the form of an aerosol or vapour to a person suffering from symptoms of nicotine dependence. Similarly, the devices of the invention may be useful in a method of treating (e.g. alleviating) the symptoms of nicotine dependence (including nicotine addiction or nicotine withdrawal). Such symptoms may include cravings for nicotine, anger/irritability, anxiety, depression, impatience, trouble sleeping, restlessness, hunger or weight gain, and/or difficulty concentrating.

When the deliverable agent is an active therapeutic agent, the delivery of that agent to the user may be intended for the treatment of a disease or condition. For the avoidance of doubt, by "treatment" we include the therapeutic treatment, as well as the symptomatic treatment, the prophylaxis, or the diagnosis, of the condition.

In embodiments in which the plant material is tobacco, suitable daily dosages of the nicotine that is to be delivered using the device may be from about 1 to about 100 mg/day. Conventional cigarettes typically contain between about 8 and 20 mg nicotine. It is preferred that the devices and cartridges disclosed herein contain an amount of nicotine that is at least equivalent to one cigarette. When the nicotine is supplied to the user in the form of replaceable cartridges (e.g. tablets, pellets or sticks), then each cartridge may contain from about 8 mg to about 20 mg of nicotine. As the devices may be capable of delivering substantially all of the nicotine held within the carrier material to the user, each cartridge or device may advantageously contain a lower amount of nicotine (e.g. from about 100 µg to about 5 mg, or preferably from about 1 mg to about 3 mg) while still being able to deliver an amount of nicotine to the user that is approximately equivalent to that inhaled when smoking a single cigarette. Higher quantities nicotine may also be held in a single device or cartridge, e.g. up to 100 mg, up to 500 mg or up to 1 g. Such devices and cartridges would be intended to be used multiple times, either over a single day or several days, before the device needs to be refilled or the cartridge needs to be refilled or replaced. Such quantities of nicotine may be used in devices, cartridges and formulation units which are used in medicine, e.g. in the treatment of nicotine dependence (e.g. nicotine addiction), treating (or alleviating) the symptoms of nicotine dependence (including nicotine addiction or nicotine withdrawal), dementia, Alzheimer's disease, Parkinson's disease, Huntington's disease, and depression. Suitable doses for the treatment of such diseases (typically *via* a nasal spray) may range from 5 to 15 mg/day. A device or cartridge according to the invention may therefore contain a sufficient amount for a single daily dose, or a fraction thereof (e.g. one half, one third or one quarter).

According to a further exemplary and non-claimed aspect of the invention, there is provided a method of treating a disease or condition selected from the group consisting of nausea and vomiting (e.g. during chemotherapy), HIV/AIDS (including improving the appetite of patients suffering from HIV/AIDS), pain, neurological problems (e.g. muscle spasms), and post-traumatic stress disorder, which method involves using an inhalation device of the present invention in which the plant material is cannabis to deliver one or more cannabinoid substances in the form of an aerosol or vapour to a person suffering from said disease or condition.

According to a yet further exemplary and non-claimed aspect of the invention, there is provided a method of suppressing hunger or thirst, or a method of treating a disease or condition selected from the group consisting of altitude sickness, fatigue and pain (e.g. pain associated with headaches, rheumatism, wounds and sores), which method involves using an inhalation device of the present invention in which the plant material is obtained from the coca plant to deliver one or more coca plant alkaloids in the form of an aerosol or vapour to a person suffering from said disease or condition.

Inhalation devices of the present invention which contain an active pharmaceutical ingredient are capable of releasing a pharmacologically effective amount of the active ingredient during normal use. By "pharmacologically effective amount", we refer to an amount of active ingredient, which is capable of conferring a desired pharmacological or therapeutic effect on a treated patient, whether administered alone or in combination with another active ingredient. Such an effect may be objective (i.e. measurable by some test or marker) or subjective (i.e. the subject gives an indication of, or feels, an effect).

More preferred compositions of the invention may be adapted (for example as described herein) to provide a sufficient dose of drug over the dosing interval (irrespective of the number of doses per unit time) to produce a desired therapeutic effect.

The amounts of active ingredients that may be employed in devices of the invention may thus be determined by the physician, or the skilled person, in relation to what will be most suitable for an individual patient. This is likely to vary with the type and severity of the condition that is to be treated, as well as the age, weight, sex, renal function, hepatic function and response of the particular patient to be treated. The above-mentioned dosages are exemplary of the average case; there can, of course, be individual instances where higher or lower dosage ranges are merited, and such are within the scope of this invention.

The devices of the invention may provide protection against intentional mechanical breakdown of the carrier material, e.g. by traditional methods such as crushing, pestle and mortar, hammering etc. due to the carrier material having a high compressive strength level at the micro-level material.

Devices of the invention, and particularly the carrier materials that are used therein, may also have the advantage that they may be prepared using established pharmaceutical processing methods and may employ materials that are approved for use in foods or pharmaceuticals or of like regulatory status.

Carrier materials that are used in the devices of the invention may also have the advantage that they may be more efficacious than, be less toxic than, be faster acting than, be more potent than, produce fewer side effects than, be more easily absorbed than, and/or have a better pharmacokinetic profile than, and/or have other useful pharmacological, physical, or chemical properties over, pharmaceutical compositions known in the prior art. This is particularly the case for embodiments in which the device of the invention, or the carrier material (e.g. in the case of replaceable cartridges) does not comprise a traditional evaporation enhancing agent such as propylene glycol, glycerine or polyethylene glycol.

The use of the carrier materials described herein (particularly chemically bonded ceramics and geopolymers) affords for the provision of removable and replaceable units to be used in conjunction with heating devices to attain acceptable levels of release of deliverable agent under heating, while minimising the risk of exposure to the stored materials within, e.g. through leakage. The carrier materials are also easily manufactured without the need for high temperature sintering, and therefore additional elements such as conductors and magnets can be incorporated into the carrier material to aid in the heating process. The ability to incorporate the plant material into the carrier material as the carrier material structure is formed also allows for greater control over the amount of deliverable agent present.

Wherever the word "about" is employed herein in the context of dimensions (e.g. values, temperatures, pressures (exerted forces), relative humidities, sizes and weights, particle or grain sizes, pore sizes, timeframes etc.), amounts (e.g. relative amounts (e.g. numbers or percentages) of particles, individual constituents in a composition or a component of a composition and absolute amounts, such as doses of nicotine, numbers of particles, etc.), deviations (from constants, degrees of degradation, etc.) it will be appreciated that such variables are approximate and as such may vary by ± 10%, for example ± 5% and preferably ± 2% (e.g. ± 1%) from the numbers specified herein.

The invention is illustrated by the following examples in which:
Figure 1 shows the weight, diameter, height and crushing strength of calcium sulphate tablets before curing, and after 25, 48 and 144 hours of post-tableting curing (n=10-11);
Figure 2 shows the nicotine applied as e-juice released from post-tableting cured ceramic tablets (n=3);
Figure 3 shows the nicotine release from post-tableting cured ceramic tablets (n=3); and
Figure 4 shows the amount of nicotine released from tobacco loaded in calcium sulfate ceramic dose units after heat treatment in a laboratory device prototype.

### Examples

### Example 1 - Post-tableting curing of ceramic tablets

Calcium sulphate alpha hemihydrate (CaS) was obtained from Bo EhrlanderAB (Sweden). 1.5 g unsieved calcium sulphate powder was weighed and filled into the dye of a modified hydraulic bench tablet press (Nike Hydraulic, Sweden) and compacted using a force of 30 MPa. The diameter of the punch was 14 mm.

### Curing

The tablets were left to cure in a heating cabinet (Termaks A/S) at 50°C, in 100 % RH. The tablets were put on a perforated plate inside a sealed desiccator with water underneath the perforated plate, and left to cure for 25, 48 or 144 hours. After the curing in the heating cabinet the tablets were left to dry for 24 hours in ambient room conditions covered with aluminium foil before analysis.

### Tablet properties

To investigate the effect of the curing process, the tablet weight, tablet dimensions and crushing strength were tested before and after the curing process. The crushing strength was performed according to the European Pharmacopeia test number 2.9.8 EP, the test was performed at a constant speed of 0.35 mm/s in a Tablet Hardness Tester from Kraemer Elektronik GmBH, Germany.

### Results

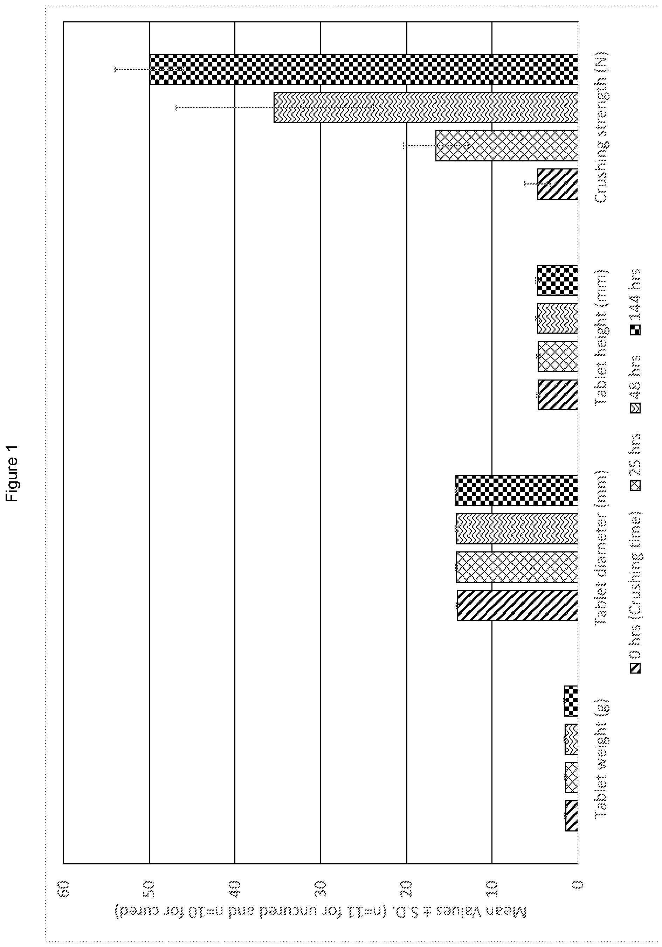

The results are shown in Figure 1.

| Curing time (hrs) | Tablet weight (g) | Tablet diameter (mm) | Tablet height (mm) | Crushing strength (N) |
|---|---|---|---|---|
| 0 | 1.4468 | 14.05 | 4.64 | 4.7 |
| 25 | 1.4746 | 14.15 | 4.64 | 16.6 |
| 48 | 1.5320 | 14.21 | 4.73 | 35.4 |
| 144 | 1.5902 | 14.23 | 4.71 | 50.0 |

Tablet weight, diameter and height were not affected by the curing process. The crushing strength increased significantly with increased time for curing process. After 144 hours, the crushing strength had reached the maximum limit of the hardness tester (50 N maximum).

### Conclusion

The increase in crushing strength demonstrate that calcium sulfate tablets have cured post-tableting.

### Example 2 - Nicotine release from post-cured ceramic tablets

Tablets obtained according to Example 1 and cured for 25 hours were loaded with a Nicotine e-juice solution (18 mg/ml) obtained from Ritchy Group Ltd via Cigoteket (Sweden).

Application of nicotine was performed by dispensing the e-juice (50 µl corresponding to 0.9 mg nicotine) onto the surface of the ceramic tablets. After application, the tablets were left to dry for 24 hours in room temperature before heat-treatment and/or analysis.

### Heating method

Oven Wilfa EMK 218 was obtained from Wilfa, (Norway). The temperature was set to approximately 200°C. The temperature was measured using an IR-thermometer from Mastech, (USA). The tablets were heated for 5 and 10 minutes respectively to let nicotine evaporate.

### Nicotine release detection

After heating, each tablet was placed in an E-flask with distilled water for extraction of the remaining nicotine. After 24 hours, 1 ml of the extract was analysed with UV-spectrophotometry at 260.5 nm. Un-heated tablets were extracted as reference at the same occasion.

The results obtained for the reference samples represent the amount of nicotine that was loaded before heat treatment. The difference in amount of nicotine detected in the heat-treated samples and the reference sample represents the amount of nicotine that has been evaporated during the heat treatment.

### Results

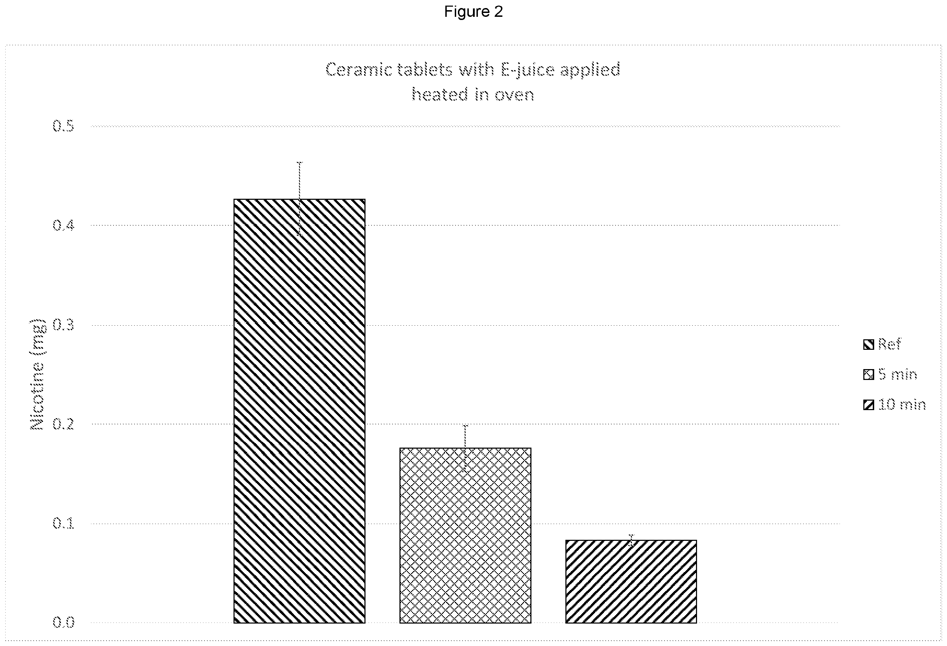

The results are shown in Figure 2. The nicotine release was 0.25 mg and 0.35 mg after 5 and 10 minutes of heating respectively, corresponding to 58% and 81% of nicotine in the reference samples.

### Conclusion

Post-tableting cured ceramic tablets can be loaded with nicotine in e-juice on the surface, and release nicotine upon heating.

### Example 3 - Nicotine release from post-tableting cured ceramic tablets

Tablets obtained according to Example 1 and cured for 48 hours were loaded with nicotine (pure) EP from BGP Healthcare pvt Ltd (India). Application of nicotine was performed by dispensing (20 µl corresponding to 20 mg nicotine) onto the surface of the ceramic tablets.

After application, the tablets were left to dry for 24 hours in room temperature before heat-treatment and/or analysis.

### Heating method

Oven Wilfa EMK 218 was obtained from Wilfa, (Norway). The temperature was set to approximately 200°C. The temperature was measured using an IR-thermometer from Mastech, (USA). The tablets were heated for 5 and 10 minutes respectively to let nicotine evaporate.

### Nicotine release detection

After heating, each tablet was placed in an E-flask with distilled water for extraction of the remaining nicotine. After 24 hours, 1 ml of the extract was analysed with UV-spectrophotometry at 260.5 nm. Un-heated tablets were extracted as reference at the same occasion.

The results obtained for the reference samples represent the amount of nicotine that was loaded before heat treatment. The difference in amount of nicotine detected in the heat-treated samples and the reference sample represents the amount of nicotine that has been evaporated during the heat treatment.

### Results

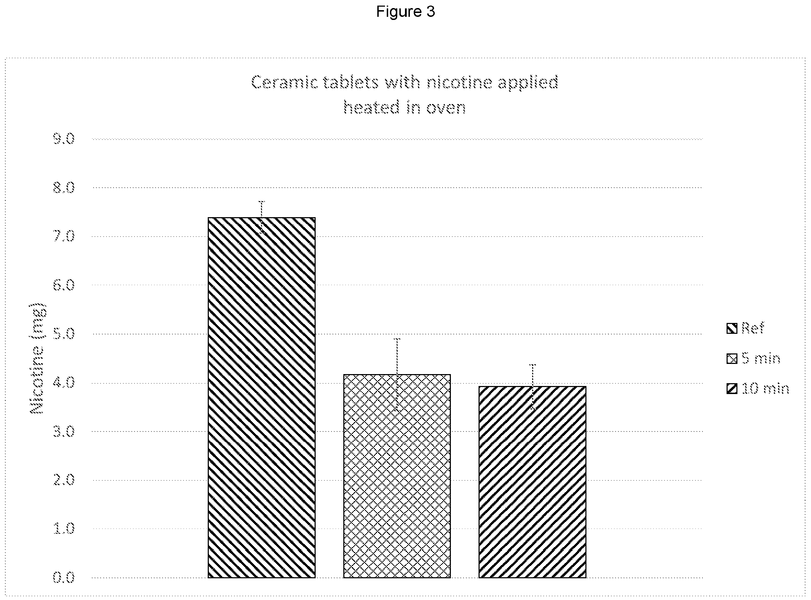

The results are shown in Figure 3. Nicotine release was demonstrated by heating ceramic tablets with nicotine applied to the tablet surface. The nicotine release was 3.2 mg and 3.5 mg after 5 and 10 minutes of heating respectively, corresponding to 43% and 47% of the reference.

### Conclusion

Post-tableting cured ceramic tablets can be loaded with pure nicotine on the surface, and release nicotine upon heating.

### Example 4 - Heating of tobacco ceramic dose units in an E-cigarette device laboratory prototype

Calcium sulfate alpha hemihydrate (CaS) were obtained from Bo Ehrlander AB (Sweden). The calcium sulphate was sieved and the fraction used was particles <100 µm. Cigarette tobacco John Silver Gray (JTI Sweden) was milled in a coffee grinder (Clatronic KSW 3306, Clatronic International GmbH, Germany). Rods were shaped in silicon rubber molds, (rods: ø 6 mm, length 12 mm).

7.5 g calcium sulphate was mixed by volume dilution with 0.26 g tobacco using a mortar and pestle. 3 ml distilled water was added to the powder mixture and mixed to form a homogenous paste, which was filled in to the molds. After the molds were filled with paste they were set to dry for at least 24 h under ambient conditions.

### Heating method

An e-cigarette laboratory device prototype (lab device prototype v2) was obtained from Devex Mekatronik AB (Sweden). The ceramic dose unit was positioned in a small test tube made of glass, which was positioned in a heat block set to 300°C.

### Nicotine release detection - simulated inhalation

The test tube was connected to a 20-ml plastic syringe via peek tubing (about 25 cm long, Ø 0.030" (0.76 mm)) with luer-lock connections. The plastic syringe was filled with 1 ml distilled water, and the piston compressed to remove most of the air. To simulate inhalation, the piston was pulled in a slow and continuous movement (about 20 ml during about 30 sec). The collected vaporized nicotine was extracted into the distilled water by shaking the syringe vigorously for at least one minute. Three simulated inhalations were performed at each time point, i.e. at 1, 3 and 5 minutes after the ceramic sample was placed in the test tube in the heat block. The nicotine content in the extracted water samples was analysed by reversed-phase HPLC-UV (detecting at 260 nm).

Reference samples were tested to determine the amount of nicotine mg/g calcium sulphate that was loaded before heat treatment.

### Results

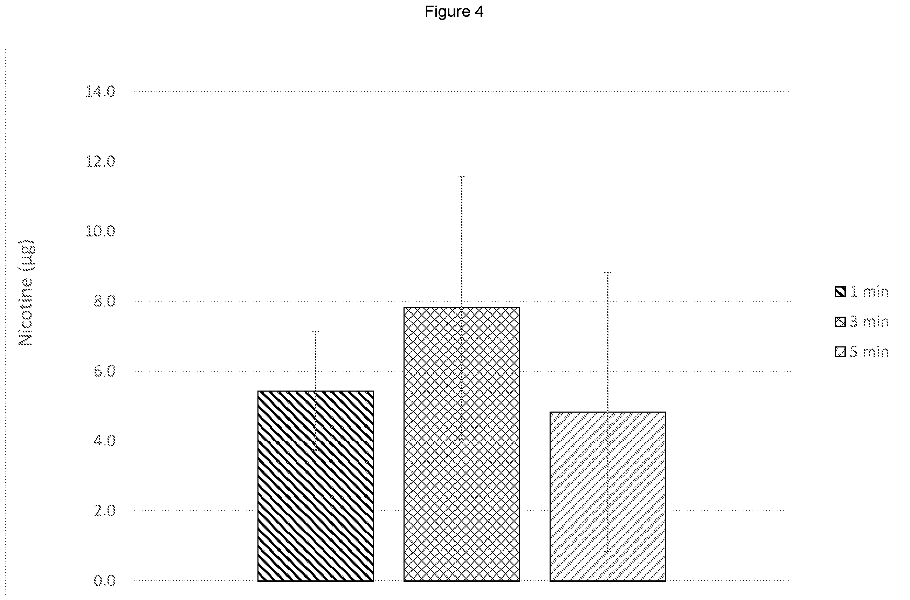

Nicotine release was demonstrated by heating ceramic dose units loaded with tobacco. Figure 4 shows the amount of nicotine released from tobacco loaded in calcium sulfate ceramic dose units after heat treatment in a laboratory device prototype. The unheated reference dose unit (for comparison) contained 0.57 mg on average (n=3).

### Conclusion

Ceramic dose units can be loaded with tobacco, and release nicotine upon heating.

### Example 5 - Heating of tobacco and nicotine ceramic dose units in an E-cigarette device laboratory prototype

Calcium sulfate alpha hemihydrate (CaS) were obtained from Bo Ehrlander AB (Sweden). Cigarette tobacco John Silver Gray (JTI Sweden) was milled using a mortar and pestle. Tablets were shaped in plastic blister pack, (width 6 mm, length 12 mm, one side flat).

5.0 g calcium sulphate was mixed with 0.17 g tobacco in a glass beaker. 0.66 g glycerol (AB Unimedic, Sweden) and 0.15 g pure nicotine (BGP Healthcare pvt, India) were mixed with 2 g distilled water and added to the powder mixture and mixed to form a homogenous paste, which was filled in to the molds. After the molds were filled with paste they were set to dry for at least 24 h in a plastic bag in room temperature.

### Heating method

An e-cigarette laboratory device prototype (lab device prototype v2) was obtained from Devex Mekatronik AB (Sweden). The ceramic dose unit was positioned in a small test tube made of glass, which was positioned in a heat block set to 300°C.

### Nicotine release detection - simulated inhalation

A 20-ml plastic syringe was placed at the opening of the test tube. To simulate inhalation, the piston was pulled in a slow and continuous movement (about 20 ml during about 20 sec). 1 ml distilled water was added to the syringe and the collected vaporized nicotine was extracted into the distilled water by shaking the syringe vigorously for at least one minute. Three simulated inhalations were performed at each time point, i.e. at 1, 2, 3, 5, 7 and 10 minutes after the ceramic sample was placed in the test tube in the heat block. The nicotine content in the water samples was analysed with UV-spectrophotometry at 260.5 nm.

Reference samples were tested to determine the amount of nicotine mg/g tablet that was loaded before heat treatment. Each tablet was placed in a plastic tube with distilled water for extraction of the nicotine. After 24 hours, 1 ml of the extract was analysed with UV-spectrophotometry at 260.5 nm.

### Results

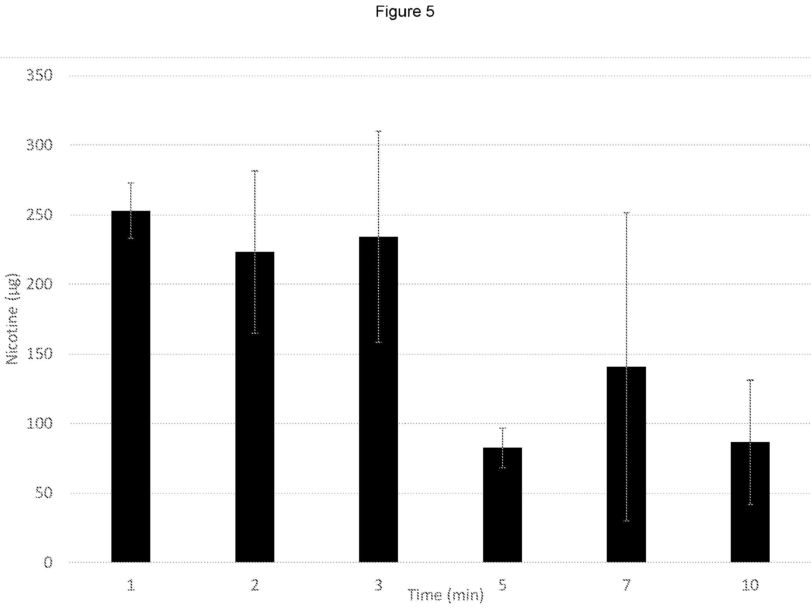

Nicotine release was demonstrated by heating ceramic dose units loaded with tobacco, nicotine and glycerol. Figure 5 shows the amount of nicotine released from the tobacco and nicotine loaded in calcium sulfate ceramic dose units after heat treatment in a laboratory device prototype. The unheated reference dose unit (for comparison) contained 13.0 mg/g on average (n=2). The average weight of the ceramic dose units was 0.3 g (n=7).

### Conclusion

Ceramic dose units can be loaded with tobacco, nicotine and glycerol. Upon heating nicotine was released.

### Example 6 - Heating of tobacco ceramic dose units post loaded with nicotine in an E-cigarette device laboratory prototype

Calcium sulfate alpha hemihydrate (CaS) were obtained from Bo Ehrlander AB (Sweden). Cigarette tobacco John Silver Gray (JTI Sweden) was milled using a mortar and pestle. Tablets were shaped in plastic blister pack, (width 6 mm, length 12 mm, one side flat).

2.5 g calcium sulphate was mixed with 0.086 g tobacco in a glass beaker. 0.33 g glycerol (AB Unimedic, Sweden) was mixed with 1 g distilled water and added to the powder mixture and mixed to form a homogenous paste, which was filled in to the molds. After the molds were filled with paste they were set to dry for at least 24 h in a plastic bag in room temperature.

Application of nicotine was performed by dispensing a mixture of pure nicotine (BGP Healthcare pvt, India) and distilled water (20 µl corresponding to 5.4 mg nicotine) onto the surface of the ceramic tablets.

### Heating method

An e-cigarette laboratory device prototype (lab device prototype v2) was obtained from Devex Mekatronik AB (Sweden). The ceramic dose unit was positioned in a small test tube made of glass, which was positioned in a heat block set to 300°C.

### Nicotine release detection - simulated inhalation

A 20-ml plastic syringe was placed at the opening of the test tube. To simulate inhalation, the piston was pulled in a slow and continuous movement (about 20 ml during about 20 sec). 1 ml distilled water was added to the syringe and the collected vaporized nicotine was extracted into the distilled water by shaking the syringe vigorously for at least one minute. Three simulated inhalations were performed at each time point, i.e. at 1, 2, 3, 5, 7 and 10 minutes after the ceramic sample was placed in the test tube in the heat block. The nicotine content in the water samples was analysed with UV-spectrophotometry at 260.5 nm.

Reference samples were tested to determine the amount of nicotine mg/g tablet. Each tablet was placed in a plastic tube with distilled water for extraction of the nicotine. After 24 hours, 1 ml of the extract was analysed with UV-spectrophotometry at 260.5 nm

### Results

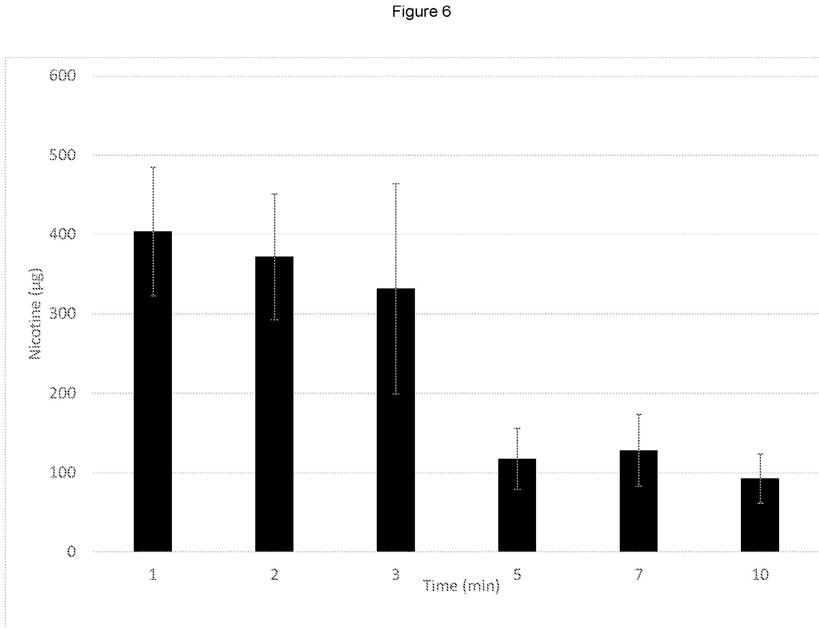

Nicotine release was demonstrated by heating ceramic dose units loaded with tobacco, nicotine and glycerol. Figure 6 shows the amount of nicotine released from the tobacco and nicotine loaded in calcium sulfate ceramic dose units after heat treatment in a laboratory device prototype. The unheated reference dose unit (for comparison) contained 15.5 mg/g on average (n=3). The average weight of the ceramic dose units was 0.3 g (n=6).

### Conclusion

Ceramic dose units can be loaded with tobacco and glycerol before curing and nicotine can be applied after curing. Upon heating nicotine was released.

## Claims

1. A device for delivering a deliverable agent in the form of an aerosol or vapour to a user, said device comprising a solid, porous carrier material, and plant material comprising the deliverable agent, wherein the carrier material is a solid, continuous three-dimensional network comprising particles of a ceramic material, which particles are bonded together to form voids and wherein the plant material is located within said voids in the carrier material, wherein the device is operable to heat the carrier material and vaporise the deliverable agent.

2. The device according to Claim 1, wherein the solid, porous carrier material has a porosity of from about 10% to about 80%.

3. The device according to Claim 1 or Claim 2, wherein:
(i) the plant material is provided in a finely divided form with an average particle size of from about 10 µm to about 3000 µηι; and/or
(ii) the plant material containing the deliverable agent is located predominantly within the voids in the carrier material.

4. The device according to any one of the preceding claims, wherein the device contains from about 0.5% to about 70% by weight plant material relative to the weight of the solid, porous carrier material, optionally wherein the device contains from about 1% to about 50% by weight plant material relative to the weight of the solid, porous carrier material.

5. The device according to any one of the preceding claims, wherein the plant material comprises plant cellular matter obtained from a single plant species or multiple plant species; optionally wherein:
(i) the plant material comprises plant cell fragments and/or plant cells; and/or
(ii) the plant material is obtained from a tobacco plant, a coca plant or a cannabis plant.

6. The device according to any one of the preceding claims, wherein the carrier material further comprises a second deliverable agent.; optionally wherein:
(i) the second deliverable agent is the same as the deliverable agent that is comprised in the plant material in the device; and/or
(ii) the second deliverable agent is located at least within pores within the carrier material.

7. The device according to any one of the preceding claims, wherein the carrier material is based on one or more chemically bonded ceramic materials, or one or more geopolymeric materials.

8. The device according to Claim 7, wherein the carrier material is selected from the list consisting of:
(i) a material obtainable by the process of reacting an aluminosilicate precursor material with an aqueous alkaline liquid; and
(ii) a calcium phosphate, a calcium sulphate, a calcium carbonate, a calcium silicate, a calcium aluminate, a magnesium carbonate, an aluminium silicate, and combinations thereof.

9. The device according to Claim 7, wherein the carrier material is (i) selected from the group consisting of calcium sulphate, a calcium phosphate, a calcium silicate, a calcium carbonate, a calcium aluminate, a magnesium carbonate, or a combination thereof, or (ii) a material obtainable by the process of reacting an aluminosilicate precursor material selected from the group consisting of kaolin, dickite, halloysite, nacrite, zeolites, illite, dehydroxylated zeolite, dehydroxylated halloysite and metakaolin with an aqueous alkaline liquid, optionally in the presence of a source of silica.

10. The device according to Claims 1 to 9, wherein the deliverable agent is nicotine, or a pharmaceutically-acceptable salt thereof; optionally wherein:
(i) the device comprises a quantity of nicotine which is not associated with the plant material; and/or
(ii) the device contains from about 8 mg to about 20 mg of nicotine.

11. The device according to any one of the preceding claims, wherein the carrier material, the plant material and the deliverable agent are provided together in a replaceable cartridge.

12. The device according to Claim 11, wherein the replaceable cartridge consists essentially of the carrier material, the plant material, the deliverable agent, and optionally particles of a conducting material and/or one or more substances selected from the group consisting of evaporation enhancing agents, flavouring agents, and taste enhancers.

13. The device according to any one of the preceding claims, further comprising a heating element operable to heat the carrier material, optionally wherein the heating element is located proximally to the carrier material.

14. A cartridge or unit dose product for use in an inhalation device, wherein the cartridge or unit dose product contains:
(i) a carrier material which is a solid, continuous three-dimensional network comprising particles of a ceramic material, which particles are bonded together to form voids; and
(ii) plant material comprising a deliverable agent; located within said voids in the carrier material;
optionally wherein the cartridge or unit dose product further contains particles of a conducting material.

15. A method of delivering a deliverable agent in the form of a vapour or aerosol to a user, which method comprises:
(a) providing an article comprising:
(i) a carrier material which is a solid, continuous three-dimensional network comprising particles of a ceramic material, which particles are bonded together to form voids; and
(ii) plant material comprising a deliverable agent located within the voids in the carrier material; and
(b) heating the carrier material to vaporise the deliverable agent.

## Patentansprüche

1. Vorrichtung zur Abgabe eines verabreichbaren Wirkstoffs in Form eines Aerosols oder Dampfs an einen Benutzer, wobei die Vorrichtung ein festes, poröses Trägermaterial und Pflanzenmaterial umfasst, das den verabreichbaren Wirkstoff umfasst, wobei das Trägermaterial ein festes, kontinuierliches dreidimensionales Netzwerk ist, das Partikel aus einem keramischen Material umfasst, wobei die Partikel unter Bildung von Hohlräumen miteinander verbunden sind und wobei sich das Pflanzenmaterial innerhalb der Hohlräume im Trägermaterial befindet, wobei die Vorrichtung dazu dient, das Trägermaterial zu erhitzen und den verabreichbaren Wirkstoff zu verdampfen.

2. Vorrichtung nach Anspruch 1, wobei das feste, poröse Trägermaterial eine Porosität von etwa 10 % bis etwa 80 % aufweist.

3. Vorrichtung nach Anspruch 1 oder Anspruch 2, wobei:
(i) das Pflanzenmaterial in feinverteilter Form mit einer durchschnittlichen Partikelgröße von etwa 10 µm bis etwa 3000 µm bereitgestellt wird; und/oder
(ii) sich das Pflanzenmaterial, das den verabreichbaren Wirkstoff enthält, überwiegend in den Hohlräumen im Trägermaterial befindet.

4. Vorrichtung nach einem der vorhergehenden Ansprüche, wobei die Vorrichtung etwa 0,5 bis etwa 70 Gew.-% Pflanzenmaterial, bezogen auf das Gewicht des festen, porösen Trägermaterials, enthält, optional wobei die Vorrichtung etwa 1 Gew.-% bis etwa 50 Gew.-% Pflanzenmaterial, bezogen auf das Gewicht des festen, porösen Trägermaterials, enthält.

5. Vorrichtung nach einem der vorhergehenden Ansprüche, wobei das Pflanzenmaterial Pflanzenzellmaterial umfasst, das von einer einzelnen Pflanzenart oder mehreren Pflanzenarten erhalten wurde; optional wobei:
(i) das Pflanzenmaterial Pflanzenzellfragmente und/oder Pflanzenzellen umfasst; und/oder
(ii) das Pflanzenmaterial aus einer Tabakpflanze, einer Kokapflanze oder einer Cannabispflanze gewonnen wird.

6. Vorrichtung nach einem der vorhergehenden Ansprüche, wobei das Trägermaterial ferner einen zweiten verabreichbaren Wirkstoff umfasst, optional wobei:
(i) der zweite verabreichbare Wirkstoff derselbe wie der verabreichbare Wirkstoff ist, der im Pflanzenmaterial in der Vorrichtung enthalten ist; und/oder
(ii) sich das zweite verabreichbare Wirkstoff zumindest innerhalb von Poren innerhalb des Trägermaterials befindet.

7. Vorrichtung nach einem der vorhergehenden Ansprüche, wobei das Trägermaterial auf einem oder mehreren chemisch gebundenen keramischen Materialien oder einem oder mehreren geopolymeren Materialien basiert.

8. Vorrichtung nach Anspruch 7, wobei das Trägermaterial ausgewählt ist aus der Liste bestehend aus:
(i) einem Material, das durch das Verfahren der Reaktion eines Aluminosilicat-Vorläufermaterials mit einer wässrigen alkalischen Flüssigkeit erhältlich ist; und
(ii) einem Calciumphosphat, einem Calciumsulfat, einem Calciumcarbonat, einem Calciumsilicat, einem Calciumaluminat, einem Magnesiumcarbonat, einem Aluminiumsilicat und Kombinationen davon.

9. Vorrichtung nach Anspruch 7, wobei das Trägermaterial (i) aus der Gruppe ausgewählt, die aus Calciumsulfat, einem Calciumphosphat, einem Calciumsilikat, einem Calciumcarbonat, einem Calciumaluminat, einem Magnesiumcarbonat oder einer Kombination davon besteht, oder (ii) ein Material ist, das durch das Verfahren der Reaktion eines Aluminosilikat-Vorläufermaterials erhältlich ist, ausgewählt aus der Gruppe bestehend aus Kaolin, Dickit, Halloysit, Nakrit, Zeolithen, Illit, dehydroxyliertem Zeolith, dehydroxyliertem Halloysit und Metakaolin, mit einer wässrigen alkalischen Flüssigkeit, optional in der Vorhandensein einer Kieselsäurequelle.

10. Vorrichtung nach den Ansprüchen 1 bis 9, wobei der verabreichbare Wirkstoff Nikotin oder ein pharmazeutisch verträgliches Salz davon ist; optional wobei:
(i) die Vorrichtung eine Nikotinmenge enthält, die nicht mit dem Pflanzenmaterial verbunden ist; und/oder
(ii) die Vorrichtung etwa 8 mg bis etwa 20 mg Nikotin enthält.

11. Vorrichtung nach einem der vorhergehenden Ansprüche, wobei das Trägermaterial, das Pflanzenmaterial und der verabreichbare Wirkstoff gemeinsam in einer austauschbaren Kartusche bereitgestellt sind.

12. Vorrichtung nach Anspruch 11, wobei die austauschbare Kartusche im Wesentlichen aus dem Trägermaterial, dem Pflanzenmaterial, dem verabreichbaren Wirkstoff und optional Partikeln eines leitenden Materials und/oder einer oder mehreren Substanzen besteht, ausgewählt aus der Gruppe bestehend aus verdunstungsfördernden Mitteln, Aromastoffen und Geschmacksverstärkern.

13. Vorrichtung nach einem der vorhergehenden Ansprüche, weiterhin umfassend ein Heizelement, das zum Erhitzen des Trägermaterials betreibbar ist, wobei sich das Heizelement optional proximal zum Trägermaterial befindet.

14. Kartusche oder Einheitsdosisprodukt zur Verwendung in einem Inhalationsgerät, wobei die Kartusche oder das Einheitsdosisprodukt Folgendes enthält:
(i) ein Trägermaterial, das ein festes, kontinuierliches dreidimensionales Netzwerk ist, das Partikel aus einem Keramikmaterial umfasst, wobei die Partikel unter Bildung von Hohlräumen miteinander verbunden sind;
und
(ii) Pflanzenmaterial, das einen verabreichbaren Wirkstoff umfasst;
innerhalb der Hohlräume im Trägermaterial angeordnet ist;
optional, wobei die Kartusche oder das Einheitsdosisprodukt ferner Partikel eines leitenden Materials enthält.

15. Verfahren zur Abgabe eines verabreichbaren Wirkstoffs in Form eines Dampfs oder Aerosols an einen Benutzer, wobei das Verfahren Folgendes umfasst:
(a) Bereitstellen eines Artikels, der Folgendes umfasst:
(i) ein Trägermaterial, das ein festes, kontinuierliches dreidimensionales Netzwerk ist, das Partikel aus einem Keramikmaterial umfasst, wobei die Partikel unter Bildung von Hohlräumen miteinander verbunden sind; und
(ii) Pflanzenmaterial, das einen verabreichbaren Wirkstoff umfasst, der sich in den Hohlräumen im Trägermaterial befindet; und
(b) Erhitzen des Trägermaterials, um den verabreichbaren Wirkstoff zu verdampfen.

## Revendications

1. Dispositif pour libérer un agent libérable sous la forme d'un aérosol ou d'une vapeur à un utilisateur, ledit dispositif comprenant un matériau support solide et poreux, et un matériau végétal comprenant l'agent libérable, dans lequel le matériau support est un réseau tridimensionnel solide et continu comprenant des particules d'un matériau céramique, lesquelles particules sont liées ensemble pour former des vides et dans lequel le matériau végétal est situé à l'intérieur desdits vides dans le matériau support, dans lequel le dispositif peut fonctionner pour chauffer le matériau support et vaporiser l'agent libérable.

2. Dispositif selon la revendication 1, dans lequel le matériau support solide et poreux a une porosité d'environ 10 % à environ 80 %.

3. Dispositif selon la revendication 1 ou la revendication 2, dans lequel :
(i) le matériau végétal est fourni sous une forme finement divisée avec une taille de particule moyenne d'environ 10 µm à environ 3 000 µm ; et/ou
(ii) le matériau végétal contenant l'agent libérable se trouve principalement dans les vides du matériau support.

4. Dispositif selon l'une quelconque des revendications précédentes, dans lequel le dispositif contient d'environ 0,5 % à environ 70 % en poids de matériau végétal par rapport au poids du matériau support solide et poreux, éventuellement dans lequel le dispositif contient d'environ 1 % à environ 50 % en poids de matériau végétal par rapport au poids du matériau support solide et poreux.

5. Dispositif selon l'une quelconque des revendications précédentes, dans lequel le matériau végétal comprend de la matière cellulaire végétale obtenue à partir d'une seule espèce végétale ou de plusieurs espèces végétales ; éventuellement dans lequel :
(i) le matériau végétal comprend des fragments de cellules végétales et/ou des cellules végétales ; et/ou
(ii) le matériau végétal est obtenu à partir d'une plante de tabac, d'une plante de coca ou d'une plante de cannabis.

6. Dispositif selon l'une quelconque des revendications précédentes, dans lequel le matériau support comprend en outre un second agent libérable ; éventuellement dans lequel :
(i) le second agent libérable est le même que l'agent libérable qui est compris dans le matériau végétal dans le dispositif ; et/ou
(ii) le second agent libérable est situé au moins à l'intérieur des pores à l'intérieur du matériau support.

7. Dispositif selon l'une quelconque des revendications précédentes, dans lequel le matériau support est à base d'un ou de plusieurs matériaux céramiques liés chimiquement, ou d'un ou de plusieurs matériaux géopolymères.

8. Dispositif selon la revendication 7, dans lequel le matériau support est sélectionné dans la liste constituée :
(i) d'un matériau pouvant être obtenu par le processus de réaction d'un matériau précurseur d'aluminosilicate avec un liquide alcalin aqueux ; et
(ii) d'un phosphate de calcium, d'un sulfate de calcium, d'un carbonate de calcium, d'un silicate de calcium, d'un aluminate de calcium, d'un carbonate de magnésium, d'un silicate d'aluminium et de combinaisons de ceux-ci.

9. Dispositif selon la revendication 7, dans lequel le matériau support est (i) choisi dans le groupe constitué du sulfate de calcium, d'un phosphate de calcium, d'un silicate de calcium, d'un carbonate de calcium, d'un aluminate de calcium, d'un carbonate de magnésium ou d'une combinaison de ceux-ci, ou (ii) un matériau pouvant être obtenu par le procédé de réaction d'un matériau précurseur d'aluminosilicate choisi dans le groupe constitué du kaolin, de la dickite, de l'halloysite, de la nacrite, des zéolites, de l'illite, de la zéolite déshydroxylée, de l'halloysite déshydroxylée et du métakaolin avec un liquide alcalin aqueux, éventuellement en présence d'une source de silice.

10. Dispositif selon les revendications 1 à 9, dans lequel l'agent libérable est la nicotine, ou un sel pharmaceutiquement acceptable de celle-ci ; éventuellement dans lequel :
(i) le dispositif comprend une quantité de nicotine qui n'est pas associée au matériau végétal ; et/ou
(ii) le dispositif contient d'environ 8 mg jusqu'à environ 20 mg de nicotine.

11. Dispositif selon l'une quelconque des revendications précédentes, dans lequel le matériau support, le matériau végétal et l'agent libérable sont fournis ensemble dans une cartouche remplaçable.

12. Dispositif selon la revendication 11, dans lequel la cartouche remplaçable est constituée essentiellement du matériau support, du matériau végétal, de l'agent libérable et éventuellement de particules d'un matériau conducteur et/ou d'une ou de plusieurs substances sélectionnées dans le groupe constitué des agents favorisant l'évaporation, des agents aromatisants et des exhausteurs de goût.

13. Dispositif selon l'une quelconque des revendications précédentes, comprenant en outre un élément chauffant utilisable pour chauffer le matériau support, éventuellement dans lequel l'élément chauffant est situé à proximité du matériau support.

14. Cartouche ou produit à dose unitaire destiné à être utilisé dans un dispositif d'inhalation, dans lequel la cartouche ou le produit à dose unitaire contient :
(i) un matériau support qui est un réseau tridimensionnel solide et continu comprenant des particules d'un matériau céramique, lesquelles particules sont liées ensemble pour former des vides ; et
(ii) du matériau végétal comprenant un agent libérable ; situé à l'intérieur desdits vides dans le matériau support ;
éventuellement dans lequel la cartouche ou le produit en dose unitaire contient en outre des particules d'un matériau conducteur.

15. Procédé de libération d'un agent libérable sous la forme d'une vapeur ou d'un aérosol à un utilisateur, lequel procédé comprend :
(a) la fourniture d'un article comprenant :
(i) un matériau support qui est un réseau tridimensionnel solide et continu comprenant des particules d'un matériau céramique, lesquelles particules sont liées ensemble pour former des vides ; et
(ii) un matériau végétal comprenant un agent libérable situé dans les vides du matériau support ; et
(b) le chauffage du matériau support pour vaporiser l'agent libérable.
